(19) Europäisches Patentamt
European Patent Office
Office européen des brevets

(11) Publication number: **0 465 716 A1**

(12) **EUROPEAN PATENT APPLICATION**

(21) Application number: **90119802.8**

(22) Date of filing: **16.10.90**

(51) Int. Cl.[5]: **C07D 513/04**, A61K 31/435, //(C07D513/04,285:00,221:00)

(30) Priority: **10.07.90 JP 180663/90**

(43) Date of publication of application: **15.01.92 Bulletin 92/03**

(84) Designated Contracting States: **AT BE CH DE DK ES FR GB IT LI NL SE**

(71) Applicant: **HOKURIKU PHARMACEUTICAL CO., LTD.**
**1-Chome, 3-14, Tatekawacho, Katsuyama-shi**
**Fukui-ken(JP)**

(72) Inventor: **Ito, Yasuo**
**11-14, Moto-machi 3-chome**
**Katsuyama-shi, Fukui-ken(JP)**
Inventor: **Kato, Hideo**
**5-8, Kentoku 3-chome**
**Fukui-ken, Fukui-ken(JP)**
Inventor: **Koshinaka, Eiichi**
**6-3, Asahi-cho 2-chome**
**Katsuyama-shi, Fukui-ken(JP)**
Inventor: **Ogawa, Nobuo**
**6-5, Ashai-cho 2-chome**
**Katsuyama-shi, Fukui-ken(JP)**
Inventor: **Yagi, Noriyuki**
**12-6-2, Inokeya**
**Katsuyama-shi, Fukui-ken(JP)**
Inventor: **Yoshida, Toshihiko**
**19-35, Otsuki, Kamishii-mura**
**Yoshida-gun, Fukui-ken(JP)**
Inventor: **Suzuki, Tomio**
**19-15, Otsuki, Kamishii-mura**
**Yoshida-gun, Fukui-ken(JP)**

(74) Representative: **Werner, Hans-Karsten, Dr. et al**
**Deichmannhaus am Hauptbahnhof**
**W-5000 Köln 1(DE)**

(54) **Optically active thiazetoquino-line-3-carboxylic acid compound, method for preparation thereof, and a pharmaceutical composition comprising the same.**

(57) An optically active thiazetoquinoline-3-carboxylic acid compound represented by the following formula (I):

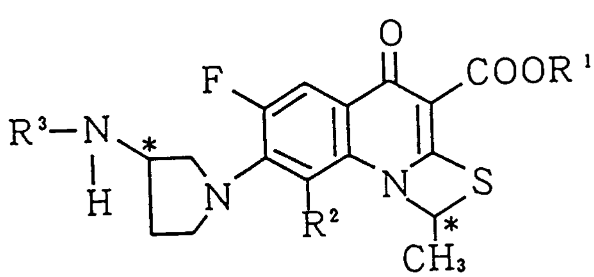

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl, or the residue of carboxylic acid ester; and C * represents an asymmetric carbon atom of S- or R-configuration, a pharmacologically acceptable salt thereof, and a method for preparing the compound are disclosed. Also disclosed are antibacterial agent, antineoplastic agent, and anti-AIDS virus agent comprising the same, a pharmaceutical composition for the treatment of bacterial infectious disease, tumor, and AIDS comprising an effective amount of the same.



Rank Xerox (UK) Business Services

BACKGROUND OF THE INVENTION

Field of the Invention

The present invention relates to a novel optically active thiazetoquinoline-3-carboxylic acid compound and a pharmacologically acceptable salt thereof which has an antibacterial activity, an antineoplastic activity, and an anti-AIDS virus activity and is useful as antibacterial agent, an antitumor agent, and anti-AIDS agent.

The present invention also relates to a method for preparing the same, a pharmaceutical composition comprising the effective amount of the same which is useful for the treatment of an infectious disease, tumor, and AIDS.

More particularly, the present invention relates to an optically active thiazetoquinoline-3-carboxylic acid compound represented by the following formula (I):

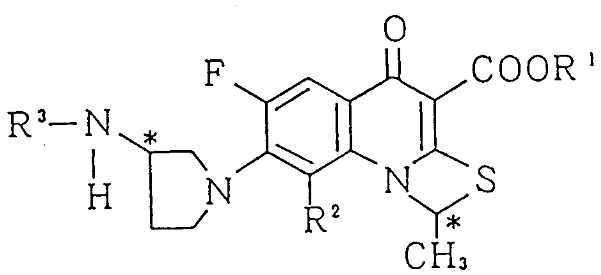

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl group, or the residue of carboxylic acid ester; and C * represents an asymmetric carbon atom of S- or R-configuration. The present invention also relates to a pharmacologically acceptable salt of compounds of formula (I), a process for preparing the same, and a pharmaceutical composition comprising the same together with a pharmaceutically acceptable carrier or coating.

Description of the Prior Art

Since the development of nalidixic acid, pyridonecarboxylic acids antibacterials have become the chief, most widely used antibacterial chemotherapeutants for the clinical treatment of an infectious diseases such as urinary tract infection, intestinal infection and cholangia infection.

Recently, norfloxacin (The Merck Index, 11th Edition, 6617), ofloxacin (The Merck Index, 11th Edition, 6688), and ciprofloxacin (The Merck Index, 11th Edition, 2315) represented by the following formula (II):

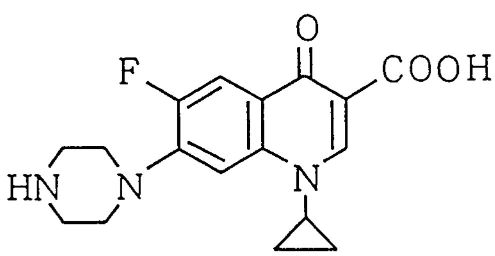

(II)

were synthesized successively and have been found to have excellent antibacterial activities and a wide antibacterial spectra compared with known synthetic antibacterials. These compounds have been developed as antibacterial agents on the market for the clinical treatment of prostatitis or otolaryngologic, internal, ophthalmologic, and dental infections as well as the above-mentioned infectious diseases.

Japanese Unexamined Patent Publication No. 107990/1988 discloses 6-fluoro-1-methyl-4-oxo-7-(1-piperazinyl)-1H,4H-[1,3]thiazeto[3,2-a]quinoline-3-carboxylic acid represented by the following formula (III):

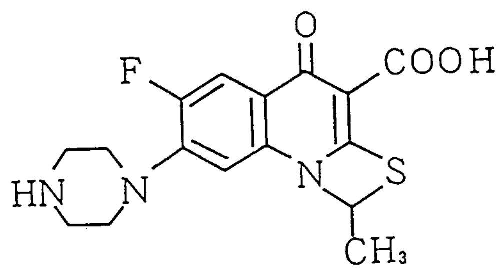

(III)

Japanese Unexamined Patent Publication No. 230584/1989 discloses 6,8-difluoro-1-methyl-4-oxo-7-(1-piperazinyl)-1H,4H-[1,3]thiazeto[3,2-a]quinoline-3-carboxylic acid represented by the following formula (IV):

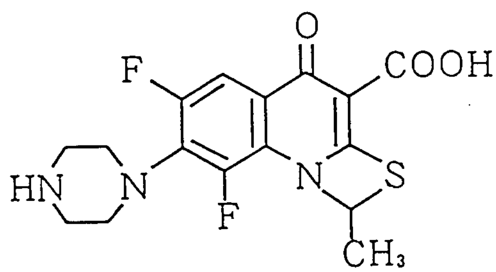

(IV)

Japanese Unexamined Patent Publication No. 107990/1988 discloses 7-(3-amino-1-pyrrolidinyl)-6-fluoro-1-methyl-4-oxo-1H,4H-[1,3]thiazeto[3,2-a]quinoline-3-carboxylic acid represented by the following formula (V):

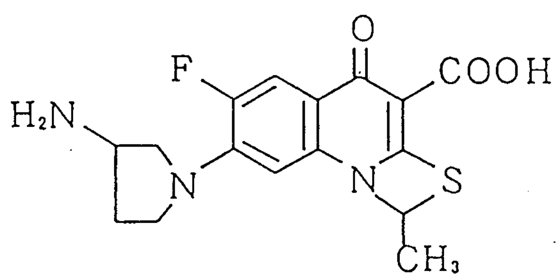

(V)

Japanese Unexamined Patent Publication No. 230584/1989 discloses 7-(3-amino-1-pyrrolidinyl)-6,8-difluoro-4-oxo-1H,4H-[1,3]thiazeto[3,2-a]quinoline-3-carboxylic acid represented by the following formula (VI):

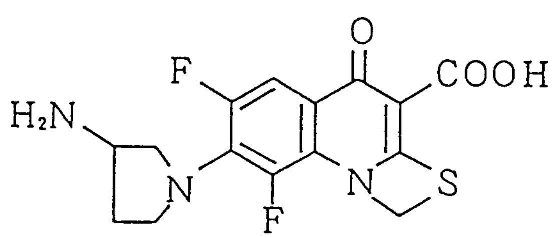

(VI)

However, none of the above documents discloses a thiazetoquinoline compound having substituents of a methyl group, an aminopyrrolidinyl group, and a halogen atom in the 1, 7, and 8 positions of the thiazetoquinoline ring, respectively. Further, a separation of the mixture of diastereoisomers having asymmetric carbon atoms in the 1-position of the thiazetoquinoline ring and in a substituent in the 7-position is not taught by any prior art.

Pyridonecarboxylic acid antibacterials have been remarkably improved since the discovery of norfloxacin, and have been found to be useful for the clinical treatment of various kinds of infectious diseases including urinary tract infection. The mode of antibacterial action of the pyridonecarboxylic acid antibacterials is believed to be an inhibitory action against DNA gyrase which is classified as a DNA topoisomerase. It is also believed that a bacterial resistance against a pyridonecarboxylic acid is not transmitted to other bacteria via plasmid DNAs, unlike other antibiotics.

Clinically, however, low-sensitive strains against the pyridonecarboxylic acids have found to be increasing, and the developed antibacterials has become ineffective for the treatment of intractable diseases such as for example a chronic infection caused by Pseudomonas aeruginosa or Gram-positive bacterial infections. Furthermore, some pyridonecarboxylic acid antibacterial agents have been revealed to cause convulsions when used in combination with certain anti-inflammatory agents.

Therefore, the above-mentioned, presently available antibacterials are insufficient from a clinical point of view. Much improvement has been longed for with respect to (1) antibacterial activities against clinically isolated resistant bacteria and (2) safety of administration, e.g., eliminating possible convulsions caused by administration with an anti-inflammatory agent.

## SUMMARY OF THE INVENTION

An object of the present invention is to provide a novel pyridonecarboxylic acid compound having excellent antibacterial activities against clinically isolated bacteria as well as against standard laboratory strains, which is low toxic and eliminates adverse reactions such as convulsions when administered in combination with an anti-inflammatory agent.

Another object of the present invention is to provide a pyridonecarboxylic acid compound having an antineoplastic activity and an anti-AIDS virus activity which is useful as an antitumor agent and anti-AIDS agent.

A further object of the present invention is to provide a pharmaceutical composition comprising said pyridonecarboxylic acid compound which is useful for the treatment of various kinds of bacterial infections.

The inventors of the present invention have conducted various studies to achieve the foregoing objects and found that the objects can be effectively attained by introducing a methyl group, an optically active aminopyrrolidinyl group, and a halogen atom to the 1, 7, and 8 positions of the thiazetoquinoline ring, respectively and separating the resulting diastereoisomers to obtain an optically active thiazetoquioline compound. The optically active compound has a remarkably increased antibacterial activity against various kinds of bacteria, in particular, against clinically isolated resistant bacteria, as compared with known thiazetoquinoline compounds. The optically active compound does not induce convulsion as used in combination with an anti-inflammatory agent, and further, it has an excellent antineoplastic activity and anti-AIDS virus activity.

According to the present invention, there is provided a novel optically active thiazetoquinoline-3-carboxylic acid compound represented by the formula (I),

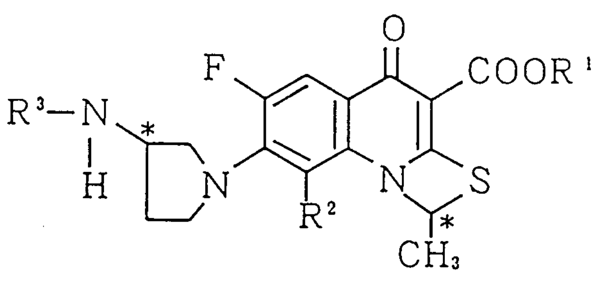

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl group, or the residue of carboxylic acid ester; and C * represents an asymmetric carbon atom of S- or R-configuration, and pharmacologically acceptable salts of the above compounds.

The present invention also provides a process for preparing the compounds of formula (I), and antibacterial agent, antitumor agent or anti-AIDS agent comprising the same.

The present invention further provides a pharmaceutical composition comprising an effective amount of the same together with a pharmaceutically acceptable carrier or coating, which is useful for the treatment of, for example, infectious disease, tumor, or AIDS .

DETAILED DESCRIPTION OF THE INVENTION

In the formula (I), examples of the lower alkyl group represented by $R^1$ include methyl, ethyl, n-propyl, isopropyl, n-butyl, sec-butyl, and tert-butyl groups; examples of the lower alkanoyl group represented by $R^3$ include formyl, acetyl, propanoyl, butyroyl, and trimethylacetyl groups; examples of the halogenated alkanoyl group represented by $R^3$ include fluoroacetyl, difluoroacetyl, trifluoroacetyl, chloroacetyl, dichloroacetyl, and trichloroacetyl groups; and examples of the residue of carboxylic acid ester represented by $R^3$ include benzyloxycarbonyl, ethoxycarbonyl, methoxycarbonyl, and tert-butoxycarbonyl groups.

Particularly preferred examples of the present invention include:
(1S, 3'S)-7-(3-amino-1-pyrrolidinyl)-6,8-difluoro-1-methyl-4-oxo-1H,4H-[1,3]thiazeto[3,2-a]quinoline-3-carboxylic acid;
(1R, 3'S)-7-(3-amino-1-pyrrolidinyl)-6,8-difluoro-1-methyl-4-oxo-1H,4H-[1,3]thiazeto[3,2-a]quinoline-3-carboxylic acid;
(1S, 3'R)-7-(3-amino-1-pyrrolidinyl)-6,8-difluoro-1-methyl-4-oxo-1H,4H-[1,3]thiazeto[3,2-a]quinoline-3-carboxylic acid;
(1R, 3'R)-7-(3-amino-1-pyrrolidinyl)-6,8-difluoro-1-methyl-4-oxo-1H,4H-[1,3]thiazeto[3,2-a]quinoline-3-carboxylic acid;
(1S, 3'S)-7-(3-amino-1-pyrrolidinyl)-8-chloro-6-fluoro-1-methyl-4-oxo-1H,4H-[1,3]thiazeto[3,2-a]quinoline-3-carboxylic acid;
(1R, 3'S)-7-(3-amino-1-pyrrolidinyl)-8-chloro-6-fluoro-1-methyl-4-oxo-1H,4H-[1,3]thiazeto[3,2-a]quinoline-3-carboxylic acid;
(1S, 3'R)-7-(3-amino-1-pyrrolidinyl)-8-chloro-6-fluoro-1-methyl-4-oxo-1H,4H-[1,3]thiazeto[3,2-a]quinoline-3-carboxylic acid;
(1R, 3'R)-7-(3-amino-1-pyrrolidinyl)-8-chloro-6-fluoro-1-methyl-4-oxo-1H,4H-[1,3]thiazeto[3,2-a]quinoline-3-carboxylic acid;
ethyl (1S, 3'S)-6,8-difluoro-1-methyl-4-oxo-7-(3-trifluoroacetylamino-1-pyrrolidinyl)-1H,4H-[1,3]thiazeto[3,2-a]-quinoline-3-carboxylate;
ethyl (1R, 3'S)-6,8-difluoro-1-methyl-4-oxo-7-(3-trifluoroacetylamino-1-pyrrolidinyl)-1H,4H-[1,3]thiazeto[3,2-a]-quinoline-3-carboxylate;
ethyl (1S, 3'R)-6,8-difluoro-1-methyl-4-oxo-7-(3-trifluoroacetylamino-1-pyrrolidinyl)-1H,4H-[1,3]thiazeto[3,2-a]-quinoline-3-carboxylate;
ethyl (1R, 3'R)-6,8-difluoro-1-methyl-4-oxo-7-(3-trifluoroacetylamino-1-pyrrolidinyl)-1H,4H-[1,3]thiazeto[3,2-a]-quinoline-3-carboxylate;
ethyl (1S, 3'S)-8-chloro-6-fluoro-1-methyl-4-oxo-7-(3-trifluoroacetylamino-1-pyrrolidinyl)-1H,4H-[1,3]thiazeto-[3,2-a]quinoline-3-carboxylate;
ethyl (1R, 3'S)-8-chloro-6-fluoro-1-methyl-4-oxo-7-(3-trifluoroacetylamino-1-pyrrolidinyl)-1H,4H-[1,3]thiazeto-[3,2-a]quinoline-3-carboxylate;
ethyl (1S, 3'R)-8-chloro-6-fluoro-1-methyl-4-oxo-7-(3-trifluoroacetylamino-1-pyrrolidinyl)-1H,4H-[1,3]thiazeto-[3,2-a]quinoline-3-carboxylate;
ethyl (1R, 3'R)-8-chloro-6-fluoro-1-methyl-4-oxo-7-(3-trifluoroacetylamino-1-pyrrolidinyl)-1H,4H-[1,3]thiazeto-[3,2-a]quinoline-3-carboxylate;
(1S, 3'S)-6,8-difluoro-1-methyl-4-oxo-7-(3-trifluoroacetylamino-1-pyrrolidinyl)-1H,4H-[1,3]thiazeto[3,2-a]-quinoline-3-carboxylic acid;
(1R, 3'S)-6,8-difluoro-1-methyl-4-oxo-7-(3-trifluoroacetylamino-1-pyrrolidinyl)-1H,4H-[1,3]thiazeto[3,2-a]-quinoline-3-carboxylic acid;
(1S, 3'R)-6,8-difluoro-1-methyl-4-oxo-7-(3-trifluoroacetylamino-1-pyrrolidinyl)-1H,4H-[1,3]thiazeto[3,2-a]-quinoline-3-carboxylic acid;
(1R, 3'R)-6,8-difluoro-1-methyl-4-oxo-7-(3-trifluoroacetylamino-1-pyrrolidinyl)-1H,4H-[1,3]thiazeto[3,2-a]-quinoline-3-carboxylic acid;
(1S, 3'S)-8-chloro-6-fluoro-1-methyl-4-oxo-7-(3-trifluoroacetylamino-1-pyrrolidinyl)-1H,4H-[1,3]thiazeto[3,2-a]-quinoline-3-carboxylic acid;
(1R, 3'S)-8-chloro-6-fluoro-1-methyl-4-oxo-7-(3-trifluoroacetylamino-1-pyrrolidinyl)-1H,4H-[1,3]thiazeto[3,2-a]-quinoline-3-carboxylic acid;
(1S, 3'R)-8-chloro-6-fluoro-1-methyl-4-oxo-7-(3-trifluoroacetylamino-1-pyrrolidinyl)-1H,4H-[1,3]thiazeto[3,2-a]-quinoline-3-carboxylic acid;
(1R, 3'R)-8-chloro-6-fluoro-1-methyl-4-oxo-7-(3-trifluoroacetylamino-1-pyrrolidinyl)-1H,4H-[1,3]thiazeto[3,2-a]-quinoline-3-carboxylic acid;
ethyl (1S, 3'S)-7-(3-amino-1-pyrrolidinyl)-6,8-difluoro-1-methyl-4-oxo-1H,4H-[1,3]thiazeto[3,2-a]quinoline-3-carboxylate;
ethyl (1R, 3'S)-7-(3-amino-1-pyrrolidinyl)-6,8-difluoro-1-methyl-4-oxo-1H,4H-[1,3]thiazeto[3,2-a]quinoline-3-carboxylate;
ethyl (1S, 3'R)-7-(3-amino-1-pyrrolidinyl)-6,8-difluoro-1-methyl-4-oxo-1H,4H-[1,3]thiazeto[3,2-a]quinoline-3-carboxylate;
ethyl (1R, 3'R)-7-(3-amino-1-pyrrolidinyl)-6,8-difluoro-1-methyl-4-oxo-1H,4H-[1,3]thiazeto[3,2-a]quinoline-3-carboxylate;
ethyl (1S, 3'S)-7-(3-amino-1-pyrrolidinyl)-8-chloro-6-fluoro-1-methyl-4-oxo-1H,4H-[1,3]thiazeto[3,2-a]-quinoline-3-carboxylate;
ethyl (1R, 3'S)-7-(3-amino-1-pyrrolidinyl)-8-chloro-6-fluoro-1-methyl-4-oxo-1H,4H-[1,3]thiazeto[3,2-a]-quinoline-3-carboxylate;
ethyl (1S, 3'R)-7-(3-amino-1-pyrrolidinyl)-8-chloro-6-fluoro-1-methyl-4-oxo-1H,4H-[1,3]thiazeto[3,2-a]-quinoline-3-carboxylate; and
ethyl (1R, 3'R)-7-(3-amino-1-pyrrolidinyl)-8-chloro-6-fluoro-1-methyl-4-oxo-1H,4H-[1,3]thiazeto[3,2-a]-quinoline-3-carboxylate;

The pharmacologically acceptable salts of the compound of the present invention represented by the above formula (I) may be acid addition salts or alkali addition salts. Examples of the acid addition salts include mineral acid salts such as for example hydrochloride, hydrobromide, hydroiodide, nitrate, sulfate, or phosphate, and organic acid salts such as for example acetate, maleate, fumarate, citrate, oxalate, malate, methanesulfonate, p-toluenesulfonate, mandelate, 10-camphorsulfonate, or tartarate. Examples of the alkali addition salts include inorganic alkali salts such as for example sodium, potassium, calcium, silver, zinc, lead, or ammonium salts, and organic alkali salts such as for example ethanolamine salt or N, N-dialkylethanolamine salt.

The thiazetoquinoline-3-carboxylic acid compound of the present invention represented by the formula (I) can be produced according to the method described below.

The optically active thiazetoquinoline compound represented by the formula (I) or a pharmacologically acceptable salt thereof may be produced by a process of the present invention comprising the steps of

(a) reacting an optically active aminopyrrolidinyl compound represented by the following formula (VII) having an asymmetric carbon atom of S- or R-configuration:

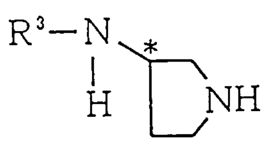

(VII)

wherein $R^3$ and $C^*$ are the same as those defined above, with racemic thiazetoquinoline compound represented by the following formula (VIII):

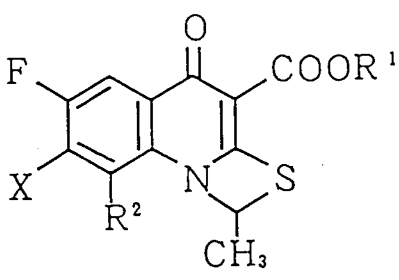

(VIII)

wherein $R^1$ and $R^2$ are the same as those defined above, and X represents a halogen atom, and followed by hydrolyzing if necessary, or chlorinating a thiazetoquinoline compound represented by the following formula (IX):

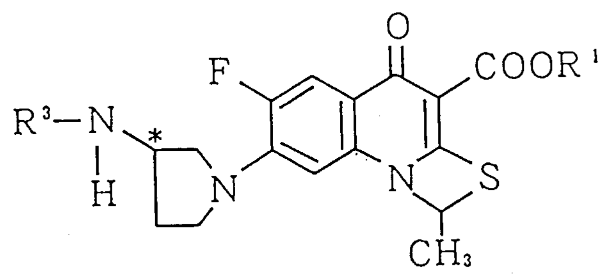

(IX)

wherein $R^1$ , $R^3$ , and C * are the same as those defined above, and followed by hydrolyzing if necessary to obtain a mixture of diasteroisomers,

(b) converting the mixture to a salt thereof, if necessary,

(c) fractionally recrystallizing or chromatographically separating the mixture of diastereomers to obtain an optically active compound, and

(d) hydrolysing the resulting optically active compound, if necessary.

The salt of the mixture may be acid addition salts or alkali addition salts. Examples of the acid addition salts include mineral acid salts such as for example hydrochloride, hydrobromide, hydroiodide, nitrate, sulfate, or phosphate, and organic acid salts such as for example acetate, maleate, fumarate, citrate, oxalate, malate, methanesulfonate, p-toluenesulfonate, mandelate, 10-camphorsulfonate, or tartarate. Examples of the alkali addition salts include inorganic alkali salts such as for example sodium, potassium, calcium, silver, zinc, lead, or ammonium salts, and organic alkali salts such as for example ethanolamine salt or N,N-dialkylethanolamine salt.

Examples of the solvent used for the fractional recrystallization include alcohols such as for example methanol, ethanol, n-propanol, isopropanol, or n-butanol; aprotic polar solvent such as for example acetonitrile, N,N-dimethylformamide, N-methylformamide, N-methyl-2-pyrrolidone, dimethyl sulfoxide, or hexamethylphosphoric triamide; aromatic hydrocarbons such as for example benzene or toluene; organic bases such as for example pyridine, picoline, lutidine, or collidine; ethers such as for example, tetrahydrofuran, 1,4-dioxane, ethyl ether, or isopropyl ether; halogenated hydrocarbons such as for example dichloromethane, chloroform, or 1,2-dichloroethane; esters such as for example ethyl formate, methyl acetate, ethyl acetate, or propyl acetate; water; and the mixture of the solvent.

Examples of the carrier used for the column chromatography include, for example, silica gel or alumina. Examples of the solvent used as an eluent include alcohols such as for example methanol, ethanol, n-propanol, isopropanol, or n-butanol; aprotic polar solvent such as for example acetonitrile, N,N-dimethylformamide, N-methylformamide, or N-methyl-2-pyrrolidone; aromatic hydrocarbons such as for example benzene or toluene; organic bases such as for example pyridine, picoline, lutidine, or collidine; ethers such as for example, tetrahydrofuran, 1,4-dioxane, ethyl ether, or isopropyl ether; halogenated hydrocarbons such as for example dichloromethane, chloroform, or 1,2-dichloroethane; esters such as for example ethyl formate, methyl acetate, ethyl acetate, or propyl acetate; hydrocarbons such as for example n-pentane, n-hexane, n-heptane, cyclopentane, or cyclohexane; water; and the mixture of the solvent.

The hydrolysis may be carried out according to a known method in the presence of an acid or an alkali. An acid such as for example hydrochloric acid, sulfuric acid, or fuming sulfuric acid may be used in an acidic hydrolysis reaction; and a base such as for example sodium hydroxide or potassium hydroxide may be used in an alkaline hydrolysis reaction. These acids or bases may be used in the form of an aqueous solution, an alcoholic solution such as for example methanolic, ethanolic, n-butanolic, sec-butanolic, or tert-butanolic solution, or a solution of an aqueous organic solvent. The hydrolysis reaction may be carried out at a temperature of from room temperature to the reflux temperature of the reaction solvent used.

The reaction of an optically active aminopyrrolidinyl compound represented by the formula (VII) with racemic thiazetoquinoline compound represented by the formula (VIII) may be carried out in the presence of or absence of a base, and followed by hydrolysis, if necessary.

Any suitable inert solvent may be used in the process of the present invention. Examples of the inert solvent include alcohols such as for example methanol, ethanol, n-propanol, isopropanol, or n-butanol; aprotic polar solvents such as for example acetonitrile, N, N-dimethylformamide, N-methyl-2-pyrrolidone, dimethyl sulfoxide, or hexamethylphospholic triamide; aromatic hydrocarbons such as for example benzene and toluene; organic bases such as for example pyridine, picoline, lutidine, and collidine; and mixtures of the above solvents.

An organic base such as for example triethylamine or 1,8-diazabicyclo[5.4.0]-7-undecene, or an inorganic base such as for example sodium carbonate or potassium carbonate may be used as the base.

The reaction may be carried out at a temperature of from an ice-cooled temperature to the reflux temperature of the reaction solvent used.

The hydrolysis may be carried out according to a known method in the presence of an acid or an alkali. An acid such as for example hydrochloric acid, sulfuric acid, or fuming sulfuric acid may be used in an acidic hydrolysis reaction; and a base such as for example sodium hydroxide or potassium hydroxide may be used in an alkaline hydrolysis reaction. These acids or bases may be used in the form of an aqueous solution, an alcoholic solution such as for example methanolic, ethanolic, n-butanolic, sec-butanolic, or tert-butanolic solution, or a solution of an aqueous organic solvent. The hydrolysis reaction may be carried out at a temperature of from room temperature to the reflux temperature of the reaction solvent used.

The 7-halogeno-thiazetoquinoline-3-carboxylic acid represented by the above general formula (VIII) can be prepared according to the following scheme, wherein X, $R^1$, and $R^2$ are the same as those defined above:

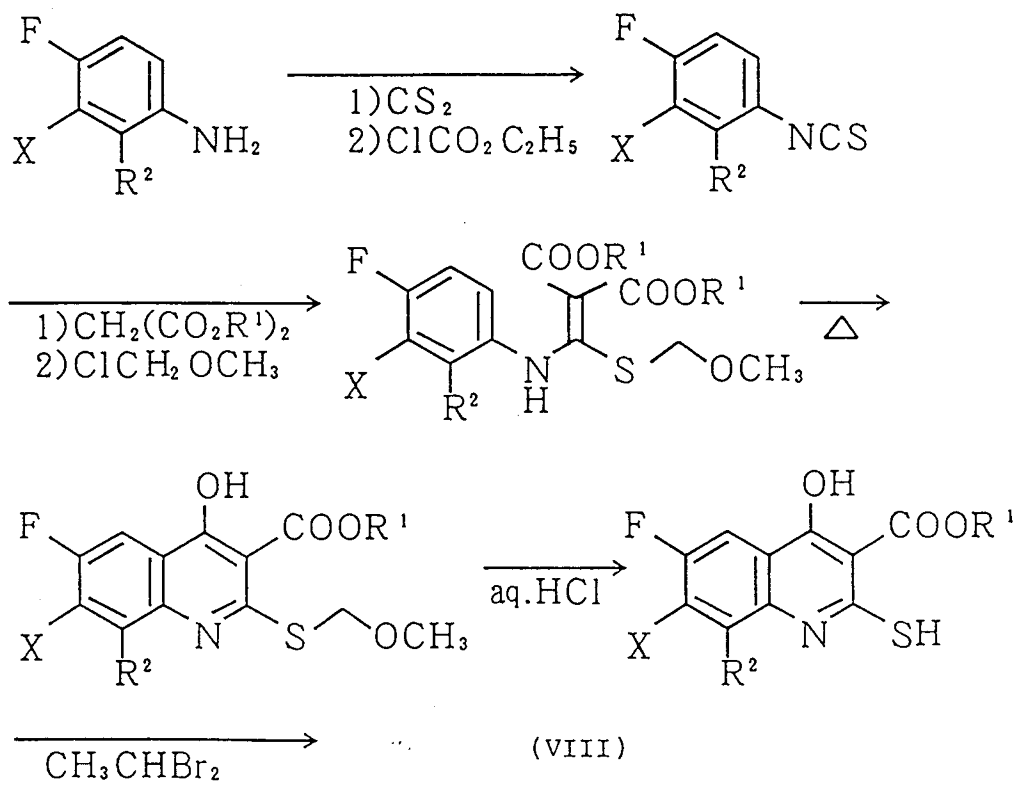


The chlorination of the compounds represented by the formula (IX) may be carried out by using chlorinating agent such as for example chlorine gas or sulfuryl chloride at a temperature of from an ice-cooled temperature to the reflux temperature of the solvent used. A solvent such as for example chloroform, dichloromethane, 1,2-dichloroethane, chlorosulfonic acid, or acetic acid may be used in the chlorination, and the chloride obtained may be hydrolyzed according to the method described above.

The optically active thiazetoquinoline-3-carboxylic acid compounds represented by the general formula (I) or pharmacologically acceptable salts thereof produced according to the method described above have potent antibacterial activity, anti-neoplastic activity, and anti-AIDS virus activity. These compounds of the present invention are also useful for the treatment of various kinds of infectious diseases, tumor, and AIDS. The optically active thiazetoquinoline-3-carboxylic acid compound of the present invention may preferably be administered orally or parenterally to a patient as a pharmaceutical composition comprising the effective amount of said thiazetoquinoline compound together with a pharmaceutically acceptable carrier or coating. The pharmaceutical composition of the present invention is useful for the treatment of, for example, bacterial infectious disease, tumor, and AIDS, which may be in the form of capsule, tablet, subtilized granule, granule, powder, or syrup for oral administration, or in the form of an injection, suppository, eye drop, eye ointment, otic solution, or dermatologic dosage form. The pharmaceutical composition of the present invention can be prepared by an ordinary method which includes the admixture of a pharmacologically and pharmaceutically acceptable carrier or coating with said thiazetoquinoline-3-carboxylic acid

compound. For the preparation of the pharmaceutical composition suitable for oral administration or suppository, the carrier or coating may comprise a diluent such as for example lactose, D-mannitol, starch, or crystalline cellulose; a disintegrant such as for example carboxymethylcellulose or calcium carboxymethylcellulose, a binder such as for example hydroxypropylcellulose, hydroxypropylmethylcellulose, or polyvinylpyrrolidone; a lubricant such as for example magnesium stearate or talc; a coating agent such as for example hydroxypropylmethylcellulose, sucrose or titanium oxide; or a base such as for example polyethyleneglycol or hard fat. The pharmaceutical composition of the present invention suitable for injection, or use as an eye drop or otic solution may comprise carriers such as, for example, a solubilizing agent or solubilizer, e.g., distilled water for injection, saline, or propylene glycol which is useful for an aqueous composition or a composition for preparing aqueous solution before use; a pH adjusting agent such as for example inorganic and organic acids or bases; tonicity agent such as for example sodium chloride, glucose, or glycerol; or stabilizer may be used. For the preparation of the phamaceutical composition suitable for eye ointment or dermatologic medicine, a carrier or coating such as for example a pharmaceutical ingredient, e.g., white petrolatum, macrogol, glycerol, or cloth which is useful for ointment, cream, or cataplasmata may be used.

Use of the pharmaceutical composition of the present invention comprises administering the composition described above internally or externally to a mammal, preferably to a human being. The dose of the pharmaceutical composition for an adult patient may generally be about from 10 to 1,000 mg per day for oral administration or about from 1 to 500 mg per day for parenteral administration, which may be increased or decreased depending on the conditions of the patient to be treated.

The effects of the compounds of the present invention are summarized in Tables 1 and 2 with respect to antibacterial spectrum against clinically isolated bacteria as well as laboratory standard strains and convulsion inducing activity, respectively, whereby ciprofloxacin hydrochloride represented by the formula (II), 6-fluoro-1-methyl-4-oxo-7-(1-piperazinyl)-1H,4H-[1,3]thiazeto[3,2-a]quinoline-3-carboxylic acid (Japanese Patent Unexamined Publication No. 107990/1988) represented by formula (III), 6,8-difluoro-1-methyl-4-oxo-7-(1-piperazinyl)-1H,4H-[1,3]thiazeto[3,2-a]quinoline-3-carboxylicacid (Japanese Patent Unexamined Publication No. 230584/1989) represented by formula (IV), 7-(3-amino-1-pyrrolidinyl)-6-fluoro-1-methyl-4-oxo-1H,4H-[1,3]thiazeto[3,2-a]quinoline-3-carboxylic acid (Japanese Patent Unexamined Publication No. 107990/1988) represented by formula (V), and 7-(3-amino-1-pyrrolidinyl)-6,8-difluoro-4-oxo-1H,4H-[1,3]thiazeto[3,2-a]-quinoline-3-carboxylic acid (Japanese Patent Unexamined publication No. 230584/1989) represented by formula (VI) were used as reference drugs A, B, C, D, and E, respectively.

A. ANTIBACTERIAL SPECTRUM

Minimum inhibitory concentrations (MIC) of test compounds were determined by the twofold agar dilution method (Chemotherapy, 29(1), 76(1981)). Overnight cultures in Mueller-Hinton broth were suspended by buffered saline gelatin. One loopful of the bacterial suspension ($10^6$ or $10^8$ colony-forming units/ml) was incubated onto the plates containing test compounds. The plates were incubated for 18 hrs at 37 °C. The MIC was defined by the lowest concentration of the drug that inhibited visible growth of bacteria. The results are summarized in Table 1.

The compounds of the present invention show excellent antibacterial activities compared with Reference Drugs against clinically isolated strains as well as standard laboratory strains.

Table 1-A Antibacterial Spectrum (Standard Laboratory Strains)

| Strain | Gram | Ex. 15 | Ex. 17 | Ex. 18 | Ex. 26 | Ref. Drug A | Ref. Drug B | Ref. Drug C | Ref. Drug D | Ref. Drug E |
|---|---|---|---|---|---|---|---|---|---|---|
| Staphylococuss aureus FDA 209PJC-1 | + | 0.025 | 0.025 | 0.006 | 0.05 | 0.20 | 0.10 | 0.10 | 0.05 | 0.10 |
| Escherichia coli NIHJ JC-2 | - | 0.012 | 0.025 | 0.006 | 0.025 | 0.025 | 0.025 | 0.05 | 0.05 | 0.10 |
| Klebsiella pneumoniae PCI-602 | - | 0.006 | 0.025 | ≦0.0015 | 0.012 | 0.012 | 0.012 | 0.025 | 0.012 | 0.05 |
| Serratia marcescens IAM 1184 | - | 0.025 | 0.05 | 0.012 | 0.05 | 0.10 | 0.05 | 0.10 | 0.10 | 0.20 |
| Pseudomonas aeruginosa IFO 3445 | - | 0.05 | 0.10 | 0.025 | 0.10 | 0.20 | 0.20 | 0.20 | 0.10 | 0.20 |
| Enterobacter cloacae 963 | - | 0.012 | 0.05 | 0.006 | 0.05 | 0.05 | 0.05 | 0.05 | 0.05 | 0.10 |

(MIC μg/ml : $10^6$ cfu/ml)

Table 1-B Antibacterial Spectrum (Clinically Isolated Strains)

| Strain | Gram | Ex. 15 | Ex. 17 | Ex. 18 | Ex. 26 | Ref. Drug A | Ref. Drug B | Ref. Drug C | Ref. Drug D | Ref. Drug E |
|---|---|---|---|---|---|---|---|---|---|---|
| Staphylococcuss aureus HPC 527 | + | 0.025 | 0.05 | 0.012 | 0.05 | 0.39 | 0.39 | 0.20 | 0.10 | 0.20 |
| Staphylococcuss aureus HPC 308 | + | 0.39 | 0.78 | 0.39 | 0.78 | 25 | 25 | 6.25 | 25 | 100 |
| Staphylococcuss aureus HPC 292 | + | 1.56 | 1.56 | 0.78 | 3.13 | 50 | 50 | 25 | >100 | >100 |
| Enterococcus faecalis HPC 984 | + | 0.10 | 0.10 | 0.05 | 0.10 | 0.39 | 0.78 | 0.39 | 0.39 | 0.78 |
| Enterococcus faecalis HPC 948 | + | 0.39 | 0.39 | 0.39 | 0.39 | 3.13 | 6.25 | 3.13 | 3.13 | 50 |
| Enterococcus faecalis HPC 975 | + | 1.56 | 1.56 | 1.56 | 3.13 | 50 | 50 | 50 | >100 | >100 |
| Enterobacter cloacae HNR 1939 | - | 0.10 | 0.10 | 0.05 | 0.10 | 0.78 | 0.20 | 0.39 | 0.39 | 0.78 |
| Enterobacter cloacae HNR 1946 | - | 0.10 | 0.20 | 0.10 | 0.20 | 0.78 | 0.39 | 0.39 | 0.78 | 1.56 |
| Enterobacter cloacae HNR 1941 | - | 0.78 | 1.56 | 1.56 | 3.13 | 25 | 12.5 | 6.25 | >100 | >100 |
| Acinetobacter calcoaceticus HNR 916 | - | 0.05 | 0.10 | 0.025 | 0.10 | 0.39 | 0.78 | 0.20 | 0.39 | 0.39 |
| Acinetobacter calcoaceticus HNR 939 | - | 0.78 | 0.78 | 0.78 | 0.78 | 6.25 | 6.25 | 3.13 | 6.25 | >100 |
| Acinetobacter calcoaceticus HNR 904 | - | 6.25 | 12.5 | 3.13 | 25 | 100 | >100 | 25 | >100 | >100 |
| Klebsiella pneumoniae HNR 858 | - | 0.10 | 0.20 | 0.05 | 0.20 | 0.78 | 0.20 | 0.39 | 0.39 | 0.78 |
| Klebsiella pneumoniae HNR 869 | - | 0.20 | 0.78 | 0.39 | 0.78 | 3.13 | 0.78 | 0.78 | 3.13 | 50 |
| Klebsiella pneumoniae HNR 828 | - | 0.78 | 1.56 | 0.78 | 1.56 | 12.5 | 1.56 | 3.13 | 50 | 50 |
| Serratia marcescens HNR 1544 | - | 0.025 | 0.05 | 0.012 | 0.05 | 0.10 | 0.05 | 0.10 | 0.10 | 0.10 |
| Serratia marcescens HNR 1792 | - | 0.39 | 1.56 | 0.78 | 1.56 | 6.25 | 6.25 | 3.13 | 50 | >100 |
| Serratia marcescens HNR 1767 | - | 1.56 | 6.25 | 3.13 | 6.25 | 50 | 50 | 12.5 | >100 | >100 |
| Pseudomonas aeruginosa HNR 1489 | - | 0.10 | 0.20 | 0.05 | 0.10 | 0.20 | 0.10 | 0.20 | 0.10 | 0.39 |
| Pseudomonas aeruginosa HNR 1472 | - | 1.56 | 3.13 | 1.56 | 6.25 | 12.5 | 6.25 | 6.25 | 50 | >100 |
| Pseudomonas aeruginosa HNR 1537 | - | 1.56 | 6.25 | 3.13 | 6.25 | 12.5 | 6.25 | 12.5 | 1.56 | >100 |

(MIC $\mu g/ml$; $10^6$ cfu/ml)

B. CONVULSION INDUCING ACTIVITY

Male mice of ddY strain, 5 weeks old, were used. Fenbufen suspended in 0.5 % carboxymethylcellulose was administered orally at a dose of 100 mg/kg. After thirty minutes, test compounds suspended in 0.5 % carboxymethylcellulose were injected intraperitoneally (i.p.) at a dose of 100 mg/kg or 300 mg/kg. From the results summarized in Table 2, it is understood that the compounds of the present invention do not cause any convulsion when administered together with fenbufen.

Table 2 Convulsion Inducing Activity

| Compound | Number of convulsion induced / Number of animals | |
|---|---|---|
| | 100 mg/kg (i.p.) | 300 mg/kg (i.p.) |
| Example 15 | 0 / 6 | 0 / 6 |
| 〃 18 | 0 / 6 | 0 / 6 |
| Reference Drug A | 4 / 6 | 6 / 6 |
| 〃 B | 1 / 6 | 6 / 6 |
| 〃 C | 6 / 6 | - |
| 〃 D | 0 / 6 | 0 / 6 |

The following References and Examples are given by way of illustration only and all not to be construed as limiting.

Reference 1

2-Chloro-3,4-difluorophenyl isothiocyanate

To a mixture of 170 g of 2-Chloro-3,4-difluoroaniline and 434 ml of triethylamine, 86.3 g of carbon disulfide was added dropwise at room temperature and the mixture was stirred for 2 weeks. The precipitates were collected by suction to give 234 g of the yellow crystals. To a suspension of 156 g of the crystals and 70 g of triethylamine in 400 ml of dichloromethane, 48 ml of ethyl chlorocarbonate was added dropwise at from -5 °C to 5 °C. After stirring for 30 minutes, ice water and 60 ml of 35 % hydrochloric acid was added dropwise to the reaction mixture at from 0 °C to 10 °C and extracted with dichloromethane. The extract was washed with water and brine, dried, and then evaporated. The residue was chromatographed on silica gel using n-hexane and the eluent was distilled to give 58 g of the desired compound as a colorless oil, b.p. 113 - 117 °C (22 mmHg).

NMR spectrum δ ($CDCl_3$)ppm: 6.90-7.27(2H, m).

In the same manner as described in Reference 1, the compounds of References 2 and 3 were prepared.

Reference 2

2,3,4-Trifluorophenyl isothiocyanate

colorless oil, b.p. 70-93 °C (25 mmHg).

NMR spectrum δ ($CDCl_3$)ppm: 6.73-7.15(2H, m).

Reference 3

3,4-Difluorophenyl isothiocyanate

colorless oil, b.p. 107-110 °C (35 mmHg).

NMR spectrum δ ($CDCl_3$)ppm: 6.67-7.33(3H, m).

Reference 4

Ethyl 8-chloro-6,7-difluoro-4-hydroxy-2-(methoxymethylthio)quinoline -3-carboxylate

To a solution of 45 g of diethyl malonate in 500 ml of abs. tetrahydrofuran, 11.2 g of 60 % sodium hydride oil suspension was added under ice cooling with stirring. After 15 minutes, 57 g of 2-chloro-3,4-difluorophenyl isothiocyanate was added to the reaction mixture at room temperature with stirring. The reaction mixture was stirred for 15 minutes and then 22.5 g of chloromethyl methyl ether was added at the same temperature.

After 15 minutes, the reaction mixture was concentrated in vacuo, and water was added to the residue. The mixture was extracted with chloroform. The extract was washed with water and brine, dried and evaporated to give 115 g of pale yellow oil. The oil was heated at from 170 °C to 175 °C under reduce pressure for 15 minutes. After cooling, the obtained precipitates were washed with ethanol and recrystallized from ethanol to give 60 g of the desired compound as colorless needles, m.p. 105-108 °C.

NMR spectrum δ ($CDCl_3$)ppm: 1.55(3H, t, J = 7Hz), 3.48(3H, s), 4.56(2H, q, J = 7Hz), 5.54(2H, s), 7.85-(1H, dd, J = 10, 8Hz).

In the same manner as described in Reference 4, the compounds of References 5 and 6 were prepared.

Reference 5

Ethyl 6,7,8-trifluoro-4-hydroxy-2-(methoxymethylthio)quinoline-3-carboxylate

Pale yellow needles (EtOH), m.p. 91-94 °C.

NMR spectrum δ (DMSO-$d_6$)ppm: 1.35(3H, t, J = 7Hz), 3.32(3H, s), 4.38(2H, q, J = 7Hz), 5.44(2H, s), 7.92(1H, ddd, J = 10.5, 8, 2Hz).

Reference 6

Ethyl 6,7-difluoro-4-hydroxy-2-(methoxymethylthio)quinoline-3-carboxylate

Colorless crystals (AcOEt), m.p. 123-127 °C.

NMR spectrum δ ($CDCl_3$)ppm: 1.54(3H, t, J = 7Hz), 3.46(3H, s), 4.56(2H, q, J = 7Hz), 5.45(2H, s), 7.55-(1H, dd, J = 11, 7Hz), 7.91(1H, dd, J = 10.5, 8.5Hz).

Reference 7

Ethyl 8-chloro-6,7-difluoro-4-hydroxy-2-mercaptoquinoline-3-carboxylate

To a solution of 15.7 g of ethyl 8-chloro-6,7-difluoro-4-hydroxy-2-(methoxymethylthio)quinoline-3-carboxylate in 125 ml of 1,4-dioxane, 95 ml of 35 % hydrochloric acid was added dropwise at 40 °C with stirring. The mixture was stirred at the same temperature for 1 hour. The reaction mixture was poured into 500 ml of water. The precipitates were collected by suction and recrystallized from acetonitrile to give 11.2 g of the desired compound as pale yellow needles, m.p. 125-130 °C (decomp.).

NMR spectrum δ ($CDCl_3$)ppm: 1.54(3H, t, J = 7Hz), 4.56(2H, q, J = 7Hz), 5.30(2H, s), 7.91(1H, dd, J = 10, 8Hz).

In the same manner as described in Reference 7, the compounds of References 8 and 9 were prepared.

Reference 8

Ethyl 6,7,8-trifluoro-4-hydroxy-2-mercaptoquinoline-3-carboxylate

Yellow crystals (DMF-EtOH), m.p. 188-190 °C (decomp.).

NMR spectrum δ (DMSO-$d_6$)ppm: 1.28(3H, t, J = 7Hz), 4. 26(2H, q, J = 7Hz), 7.83(1H, ddd, J = 11, 7.5, 2.5Hz).

Reference 9

Ethyl 6,7-difluoro-4-hydroxy-2-mercaptoquinoline-3-carboxylate

Yellow needles (DMF-EtOH), m.p. 201-203 °C (decomp.).

NMR spectrum δ (DMSO-$d_6$)ppm: 1.29(3H, t, J=7.5Hz), 4.27(2H, q, J=7.5Hz), 7.53(1H, dd, J=11.5, 7.5Hz), 8.00(1H, dd, J=11, 8. 5Hz).

Reference 10

Ethyl 8-chloro-6,7-difluoro-1-methyl-4-oxo-1H,4H-[1,3]thiazeto [3,2-a]quinoline-3-carboxylate

To a suspension of 13.7 g of 1,1-dibromoethane, 5.7 g of potassium iodide and 9.5 g of potassium carbonate in 60 ml of N,N-dimethylformamide, a solution of 11.1 g of ethyl 8-chloro-6,7-difluoro-4-hydroxy-2-mercaptoquinoline-3-carboxylate in 240 ml of N,N-dimethylformamide was added dropwise at from 110 °C to 115 °C during 2 hours with stirring. After stirring for 30 minutes at the same temperature, the reaction mixture was concentrated in vacuo and water was added to the residue. The precipitates were collected by suction and chromatographed on silica gel using chloroform. The eluent was recrystallized from a mixture of chloroform and diethyl ether to give colorless needles, m.p. 191-194 °C.

NMR spectrum δ ($CDCl_3$)ppm: 1.41(3H, t, J=7Hz), 2.27(3H, d, J=6Hz), 4.39(2H, q, J=7Hz), 6.33(1H, q, J=6Hz), 8.22(1H, dd, J=10, 8Hz).

In the same manner as described in Reference 10, the compounds of References 11 and 12 were prepared.

Reference 11

Ethyl 6,7,8-trifluoro-1-methyl-4-oxo-1H,4H-[1,3]thiazeto[3,2-a] quinoline-3-carboxylate

Colorless needles (DMF), m.p. 206-210 °C.

NMR spectrum δ (DMSO-$d_6$)ppm: 1.27(3H, t, J=7Hz), 2.06(3H, dd, J=6, 3Hz), 4.21(2H, q, J=7Hz), 6.26(1H, qd, 7=6, 2Hz), 7.84(1H, ddd, J=10.5, 7.5, 2.5Hz).

Reference 12

Ethyl 6,7-difluoro-1-methyl-4-oxo-1H,4H-[1,3]thiazeto[3,2-a] quinoline-3-carboxylate

Colorless needles (DMF), m.p. 197-199 °C.

NMR spectrum δ (DMSO-$d_6$)ppm: 1.27(3H, t, J=7.5Hz), 2.08(3H, d, J=6.5Hz), 4.19(2H, q, J=7.5Hz), 6.12(1H, q, J=6.5Hz), 7.67(1H, dd, J=11, 6.5Hz), 7.94(1H, dd, J=11, 8.5Hz).

Reference 13

Ethyl 6-fluoro-1-methyl-4-oxo-7-((R)-3-trifluoroacetylamino-1-pyrrolidinyl)-1H,4H-[1,3]thiazeto[3,2-a]quinoline-3-carboxylate

A mixture of 30.0 g of ethyl 6,7-difluoro-1-methyl-4-oxo-1H,4H -[1,3]thiazeto[3,2-a]quinoline-3-carboxylate, 25.3 g of (R)-3-trifluoroacetylaminopyrrolidine hydrochloride, [ $\alpha$ ] $^{20}_{D}$ + 26.6 ° (c=1,MeOH)] and 32.3 g of 1,8-diazabicyclo[5.4.0]-7-undecene in 240 ml of N,N-dimethylformamide was stirred at room temperature for 63 hours. The reaction mixture was concentrated in vacuo, and 50 ml of water was added to the residue. The crystals ware collected by suction and recrystallized from a mixture of N,N-dimethylformamide and ethanol to give 32.0 g of the desired compound as colorless needles, m.p. 262-265 °C.

NMR spectrum δ (DMSO-$d_6$) ppm: 1.25(3H, t, J=7Hz), 1.81-2.45 (2H, m), 2.04(3H, d, J=6Hz), 3.36-4.00(4H, m), 4.17(2H, q, J=7Hz), 4.32-4.69 (1H, m), 6.11(1H, q, J=6Hz), 6.31(1H, d, J=7.5Hz), 7.61(1H, d, J=15Hz), 9.44-9.76(1H, m).

In the same manner as described in Reference 13, the compounds of References from 14 to 16 were prepared.

Reference 14

Ethyl 6-fluoro-1-methyl-4-oxo-7-((S)-3-trifluoroacetylamino-1-pyrrolidinyl)-1H,4H-[1,3]thiazeto[3,2-a]quinoline-3-carboxylate

The desired compound was prepared using (S)-3-trifluoroacetylaminopyrrolidine hydrochloride [[ $\alpha$ $]_D^{20}$ - 28.6° (c = 1, MeOH)].

Pale gray crystals ($CHCl_3$ -EtOH), m.p. 259-261 °C (decomp.).

NMR spectrum $\delta$ (DMSO-$d_6$)ppm: 1.25(3H, t, J = 7Hz), 1.87-2.45 (2H, m), 2.04(3H, d, J = 6Hz), 3.36-4.64-(5H, m), 4.17(2H, q, J = 7Hz), 6.12(1H, q, J = 6Hz), 6.33(1H, d, J = 7.5Hz), 7.62(1H, d, J = 15Hz), 9.47-9.73-(1H, m).

Reference 15

Ethyl 7[(S)-3-(tert-butoxycarbonylamino)-1-pyrrolidinyl]-6-fluoro-1-methyl-4-oxo-1H,4H-[1,3]thiazeto[3,2-a]-quinoline-3-carboxylate

The desired compound was prepared using (S)-3-(tert-butoxycarbonylamino)pyrrolidine [[ $\alpha$ $]_D^{20}$ - 21.6° (c = 1, EtOH)].

Colorless prisms ($CH_3CN$), m.p. 242-245 °C (decomp.).

NMR spectrum $\delta$ (DMSO-$d_6$)ppm: 1.25(3H, t, J = 7Hz), 1.40(9H, s), 1.80-2.20(2H, m), 2.04(3H, d, J = 6Hz), 3.10-3.84(5H, m), 4.17(2H, q, J = 7Hz), 6.12(1H, q, J = 6Hz), 6.29(1H, d, J = 8Hz), 7.00(1H, br), 7.60-(1H, d, J = 15Hz).

Reference 16

Ethyl 7-[(R)-3-(tert-butoxycarbonylamino)-1-pyrrolidinyl]-6-fluoro-1-methyl-4-oxo-1H,4H-[1,3]thiazeto[3,2-a]-quinoline-3-carboxylate

The desired compound was prepared using (R)-3-(tert-butoxycarbonylamino)pyrrolidine [[ $\alpha$ $]_D^{20}$ + 21.4° (c = 1, EtOH)].

Pale red needles ($CH_3CN$), m.p. 242-245 °C (decomp.).

NMR spectrum $\delta$ (DMSO-$d_6$)ppm: 1. 25(3H, t, J = 7Hz), 1.40(9H, s), 1.80-2.20(2H, m), 2.03(3H, d, J = 6Hz), 3.10-3.85(5H, m), 4.17(2H, q, J = 7Hz), 6.12(1H, q, J = 6Hz), 6.28(1H, d, J = 7.5Hz), 6.98(1H, br), 7.60(1H, d, J = 15Hz).

Reference 17

Ethyl 8-chloro-6-fluoro-1-methyl-4-oxo-7- ((S)-3-trifluoroacetylamino-1-pyrrolidinyl)-1H,4H-[1,3]thiazeto[3,2-a]quinoline-3-carboxylate

To a suspension of 3.81 g of ethyl 6-fluoro-1-methyl-4-oxo-7 -((S)-3-trifluoroacetylamino-1-pyrrolidinyl)-1H,4H-[1,3]thiazeto [3,2-a]quinoline-3-carboxylate in 150 ml of dichloromethane, 0.72 ml of sulfuryl chloride was added dropwise at room temperature with stirring. After 10 minutes, the reaction mixture was washed with water four times, dried and evaporated. The residue was chromatographed on silica gel using chloroform - methanol (30:1) as an eluent to give 2.77 g of the desired compound, which was recrystallized from a mixture of ethyl acetate and isopropyl ether to give pale yellow crystals, m.p. 153-156 °C.

IR spectrum $\nu$ (KBr) $cm^{-1}$ : 1710, 1602.

NMR spectrum $\delta$ ($CDCl_3$)ppm: 1.40(3H, t, J = 7Hz), 1.88-2.60(2H, m), 2.21, 2.22(total 3H, each d, J = 6Hz), 3.16-4.82(5H, m), 4.36, 4.37 (total 3H, each q, J = 7Hz), 6.34, 6.35(total 1H, each q, J = 6Hz), 7.00 -7. 52(1H, m), 7.90, 7.97(total 1H, each d, J = 13Hz).

The product obtained was a mixture of diastereoisomers containing isomer A (retetion time: 15.4 min.) and isomer B (retetion time: 17.5 min.) in the ratio of about 53:47 analyzed under the following HPLC conditions:

| | |
|---|---|
| COLUMN: | SUMIPAX OA-3100 $\phi$ 4.6mm $\times$ 250mm |
| Carrier: | methanol |
| Flow rate: | 0.5 ml/min. |
| Detector: | UV spectrometer (287 nm) |

Reference 18

Ethyl 8-chloro-6-fluoro-1-methyl-4-oxo-7-((S)-3-trifluoroacetylamino-1-pyrrolidinyl)-1H,4H-[1,3]thiazeto[3,2-a]-quinoline-3-carboxylate

To a solution of 1.50 g of ethyl 6-fluoro-1-methyl-4-oxo-7-((S)-3-trifluoroacetylamino-1-pyrrolidinyl)-1H,4H-[1,3]thiazeto [3,2-a]quinoline-3-carboxylate in 4.0 ml of chlorosulfonic acid, a trace of iodine was added, and the mixture was bubbled with chlorine gas for 1 hour under ice cooling with stirring. The reaction mixture was poured into ice water and was extracted with chloroform. The extract was washed with aqueous potassium carbonate and water, dried and evaporated. The residue was chromatographed on silica gel using chloroform-methanol (50:1) as an eluent to give 1.61 g of the desired compound, which was recrystallized from a mixture of ethyl acetate and isopropyl ether. The obtained crystals were consistent with that of Reference 17.

In the same manner as described in References 17 and 18, the compounds of References 19 and 20 were prepared.

Reference 19

Ethyl 7-[(S)-3-(tert-butoxycarbonylamino)-1-pyrrolidinyl]-8-chloro-6-fluoro-1-methyl-4-oxo-1H,4H-[1,3]-thiazeto[3,2-a]quinoline-3-carboxylate

Colorless needles ($CH_3CN$), m.p. 115-118 °C.

IR spectrum $\nu$ (KBr)$cm^{-1}$ : 1716, 1602.

NMR spectrum $\delta$ (DMSO-$d_6$) ppm: 1.26(3H, t, J=7Hz), 1.40(9H, s), 1.58-2.44(2H, m), 2.11(3H,d, J=6Hz), 3.14-3.99 (5H, m), 4.20(2H, q, J=7Hz), 6.46(1H, q, J=6Hz), 6.90-7.11(1H, br), 7.73(1H, d, J=14Hz).

Specific rotation $[\alpha]_D^{20}$ +240.6° (c=0.1, $CHCl_3$)

Reference 20

Ethyl 7-[(R)-3-(tert-butoxycarbonylamino)-1-pyrrolidinyl]-8-chloro-6-fluoro-1-methyl-4-oxo-1H,4H-[1,3]-thiazeto[3,2-a]quinoline-3-carboxylate

Colorless crystals (AcOEt-$Et_2O$), m.p. 174-177 °C (decomp.).

IR spectrum $\nu$ (KBr)$cm^{-1}$: 1710, 1600.

NMR spectrum $\delta$ ($CDCl_3$) ppm: 1.41 (3H, t, J=7Hz), 1.46, 1.47 total 9H, each s), 1.60-2.55(2H, m), 2.21-(3H, d, J=6Hz), 3.02-4.93(5H,m), 4.38(2H, q, J=7Hz), 6.36(1H, q, J=6Hz), 8.01(1H, d, J=13Hz).

Reference 21

Ethyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate

A mixture of 1.34 g of ethyl 6,7,8-trifluoro-1-methyl-4-oxo-1H,4H-[1,3]thiazeto[3,2-a]quinoline-3-carboxylate, 2.66 g of (S)-3-trifluoroacetylaminopyrrolidine hydrochloride [[ $\alpha$ $]_D^{20}$ -28.6 ° (c=1, MeOH)], 1.80 g of 1,8-diazabicyclo[5.4.0]-7-undecene and 30 ml of N,N-dimethylformamide was stirred at room temperature for 5 hours. The reaction mixture was concentrated in vacuo, and water was added to the residue. The precipitates were collected by suction and were chromatographed on silica gel using chloroform-methanol (8:2) to give the desired compound, which was recrystallized from methanol to give 0.80 g of pale yellow needles, m.p. 219-224 °C.

IR spectrum $\nu$ (KBr)$cm^{-1}$ : 1722, 1602.

NMR spectrum $\delta$ (DMSO-$d_6$)ppm: 1.26 (3H, t, J=7Hz), 1.84-2.40 (2H, m), 2.03(3H, dd, J=6,3Hz), 3.46-4.60(5H, m), 4.18(2H, q, J=7Hz), 6.14(1H, dd, J=6, 2.5Hz), 7.48(1H, dd, J=14.5, 1.5Hz), 9.42-9.68(1H, br).

Specific rotation $[\alpha]_D^{20}$ -121.5° (c=0.1, $CHCl_3$)

In the same manner as described in Reference 21, the compounds of Reference 22 to 30 were prepared.

Reference 22

Ethyl 7-[(S)-3 -(tert-butoxycarbonylamino)-1-pyrrolidinyl]-6,8-difluoro-1-methyl-4-oxo-1H,4H-[1,3]thiazeto[3,2-a]quinoline-3-carboxylate

The desired compound was prepared using (S)-3-(tert-butoxycarbonylamino)pyrrolidine [[ α $]_D^{20}$ -21.6 ° (c = 1, EtOH)].

Pale yellow crystals ($CHCl_3$ -$Et_2O$), m.p. 179-180 °C.

IR spectrum ν (KBr)$cm^{-1}$ : 1714, 1610.

NMR spectrum δ ($CDCl_3$)ppm: 1.39(3H, t, J = 7Hz), 1.46(9H, s), 1.70-2.32(2H, m), 2.09(3H, dd, J = 6, 3Hz), 3.40-4.40(5H, m), 4.36(2H, g, J = 7Hz), 4.90-5.20(1H, br), 5.80-6.10(1H, m), 7.69(1H, dd, J = 14.5, 2Hz).

Specific rotation [ α $]_D^{20}$ + 81.7 ° (c = 0.1, $CHCl_3$)

Reference 23

Ethyl 7-[(R)-3-(tert-butoxycarbonylamino)-1-pyrrolidinyl]-6,8-difluoro-1-methyl-4-oxo-1H,4H-[1,3]thiazeto[3,2-a]quinoline-3-carboxylate

The desired compound was prepared using (R)-3-(tert-butoxycarbonylamino)pyrrolidine [[ α $]_D^{20}$ + 21.4 ° (c = 1, EtOH)].

Pale yellow crystals ($CHCl_3$ -$Et_2O$), m.p. 174-176 °C.

IR spectrum ν (KBr)$cm^{-1}$ : 1714, 1608.

NMR spectrum δ ($CDCl_3$)ppm: 1.39(3H, t, J = 7Hz), 1.46(9H, s), 1.76-2.34(2H, m), 2.10(3H, dd, J = 6, 3Hz), 3.40-4.40(5H, m), 4.36(2H, q, J = 7Hz), 4.80-5.10(1H, br), 5.80-6.10(1H, m), 7.73(1H, dd, J = 14.5, 2Hz).

Reference 24

Ethyl 8-chloro-6-fluoro-1-methyl-4-oxo-7-((S)-3-trifluoroacetylamino-1-pyrrolidinyl)-1H,4H-[1,3]thiazeto[3,2-a]-quinoline-3-carboxylate

The desired compound was prepared using (S)-3-trifluoroacetylaminopyrrolidine hydrochloride [[ α $]_D^{20}$ -28.6 ° (c = 1, MeOH)].

The obtained compound was consistent with that of Reference 17.

Reference 25

Ethyl 7-[(S)3-(tert-butoxycarbonylamino)-1-pyrrolidinyl]-8-chloro-6-fluoro-1-methyl-4-oxo-1H,4H-[1,3]thiazeto-[3,2-a]quinoline-3-carboxylate

The desired compound was prepared using (S)-3-(tert-butoxycarbonylamino)pyrrolidine [[ α $]_D^{20}$ -21.6 ° (c = 1, EtOH)].

The obtained compound was consistent with that of Reference 19.

Reference 26

Ethyl 7-[(R)-3-(tert-butoxycarbonylamino)-1-pyrrolidinyl]-8-chloro-6-fluoro-1-methyl-4-oxo-1H,4H-[1,3]-thiazeto[3,2-a]quinoline-3-carboxylate

The desired compound was prepared using (R)-3-(tert-butoxycarbonylamino)pyrrolidine [[ α $]_D^{20}$ + 21.4 ° (c = 1, EtOH)].

The obtained compound was consistent with that of Reference 20.

Reference 27

Ethyl 6,8-difluoro-1-methyl-4-oxo-7-((R)-3-trifluoroacetylamino-1-pyrrolidinyl)-1H,4H-[1,3]thiazeto[3,2-a]-quinoline-3-carboxylate

The desired compound was prepared using (R)-3-trifluoroacetylaminopyrrolidine hydrochloride [[ α $]_D^{20}$ 26.6 ° (c = 1, MeOH)].

Colorless needles (1,4-dioxane), m.p. 219-224 °C.

IR spectrum ν (KBr)$cm^{-1}$ : 1722, 1674, 1636

NMR spectrum δ ($CDCl_3$)ppm: 1.36, 1.38(total 3H, each d, J=7Hz), 1.86-2.30(2H, m), 2.02, 2.04(total 3H, d, J=6Hz), 3.51-4.17(4H, m), 4.28, 4.32(total 2H, each q, J=7Hz), 4.60, 4.73(total 1H, br-s), 5.70-6.07-(1H, m), 7.41, 7.51(total 1H, each d, J=14Hz), 8.57, 8.88(total 1H, br-s).

Reference 28

Propyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate

The desired compound was prepared using (S)-3-trifluoroacetylaminopyrrolidine hydrochloride [[ $\alpha$ $]_D^{20}$ -28.6 ° (c=1, MeOH)].

Colorless crystals (EtOH), m.p. 208-211 ° C

IR spectrum ν (KBr)$cm^{-1}$ : 1718, 1636.

NMR spectrum δ ($CDCl_3$)ppm: 1.03(3H, t, J=7Hz), 1.45-2.40(7H, m), 3.30-4.90(7H, m), 5.60-6.10(1H, m), 7.39, 7.49(total 1H, each dd, J=14, 2Hz).

Reference 29

Isopropyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate

The desired compound was prepared using (S)-3-trifluoroacetylaminopyrrolidine hydrochloride [[ $\alpha$ $]_D^{20}$ -28.6° (c=1, MeOH)].

Colorless prisms (EtOH), m.p. 235-236.5 ° C (decomp.).

IR spectrum ν (KBr)$cm^{-1}$ : 1718, 1664, 1638

NMR spectrum δ ($CDCl_3$)ppm: 1.32, 1.36(total 6H, each d, J=6.5Hz), 1.99, 2.01(total 3H, each dd, J=6, 2.5Hz), 2.05-2.33(2H, m), 3.49-4.14(4H, m), 4.53-4.78(1H, m), 5.17, 5.19(total 1H, each heptet, J=6.5Hz), 5.76, 5.92(total 1H, each qd, J=6, 3.5Hz), 7.30, 7.42(total 1H, each dd, J=14.5, 1.5Hz), 9.01, 9.31(total 1H, each d, J=7Hz).

Reference 30

Butyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate

The desired compound was prepared using (S)-3-trifluoroacetylaminopyrrolidine hydrochloride [[ $\alpha$ $]_D^{20}$ -28.6° (c=1, MeOH)].

Colorless needles (iso-PrOH), m.p. 111-112 ° C.

IR spectrum ν (KBr)$cm^{-1}$ : 1716, 1636

NMR spectrum δ ($CDCl_3$)ppm: 0.97, 0.98(total 3H, each t, J=7.5Hz), 1.38-1.58(2H, m), 1.60-1.86(2H, m), 2.00, 2.04(total 2H, each dd, J=6.5, 2.5Hz), 2.09-2.34(2H, m), 3.52-4.40(6H, m), 4.53-4.79(1H, m), 5.80, 5.98(total 1H, each qd, J=6.5, 4Hz), 7.44, 7.49(total 1H, each dd, J=14.5, 1.5Hz), 8.68, 8.77(total 1H, each d, J=7.5Hz).

Reference 31

Ethyl 8-chloro-6-fluoro-1-methyl-4-oxo-7-((R)-3-trifluoroacetylamino -1-pyrrolidinyl)-1H,4H-[1,3]thiazeto[3,2-a]quinoline-3-carboxylate

To a suspension of 47.3 g of ethyl 6-fluoro-1-methyl-4-oxo-7-((R)- 3-trifluoroacetylamino-1-pyrrolidinyl)-1H,4H-[1,3]thiazeto[3,2-a]quinoline-3-carboxylate in 237 ml of dichloromethane, 9.64 ml of sulfuryl chloride was added dropwisely at room temperature with stirring. After 10 minutes, the reaction mixture was washed with water four times, dried and evaporated. The residue was chromatographed on silica gel using a mixture of dichloromethane-ethyl acetate (2:1) as an eluent to give 49.1 g of the desired compound, which was recrystallized from isopropanol to give pale yellow crystals, m.p. 152-156 ° C.

NMR spectrum δ ($CDCl_3$) ppm: 1.38(3H, t, J=7Hz), 2.18,2.21(total 3H, each d, J=6Hz), 1.89-2.69(2H, m), 3.19-4.87(5H, m), 4.33,4.35(total 2H, each q, J=7Hz), 6.32,6.34(total 1H, each q, J=6Hz), 7.74,7.89(total 1H, each d, J=13.5Hz).

Example 1

Ethyl 8-chloro-6-fluoro-1-methyl-4-oxo-7-((S)-3-trifluoroacetylamino-1-pyrrolidinyl)-1H,4H-[1,3]thiazeto[3,2-a]-quinoline-3-carboxylate (isomer A)

Ethyl 8-chloro-6-fluoro-1-methyl-4-oxo-7-((S)-3-trifluoroacetylamino-1-pyrrolidinyl)-1H,4H-[1,3]thiazeto-[3,2-a]quinoline-3-carboxylate (129 g) which is a diastereomeric mixture of isomers A and B was dissolved in 400 ml of hot isopropanol. The insoluble materials were filtered off. The filtrate was cooled with ice water bath for 45 minutes, the precipitates were collected by suction to give 20.3 g of pale yellow crystals enriched with isomer A. After the mother liquor was kept to stand, the precipitates were collected by suction to give 93.7 g of yellow crystals, which were dissolved in 350 ml of hot isopropanol, and cooled with ice water bath. The precipitates were collected by suction to give 30.5 g of colorless crystals. The crystals obtained above were combined and recrystallized from 350 ml of isopropanol to give 36.6 g of the desired compound as colorless needles, m.p. 193-194 °C (isomer A).

IR spectrum $\nu$ (KBr)$cm^{-1}$ : 1712, 1672, 1602.

NMR spectrum $\delta$ ($CDCl_3$)ppm: 1.38(3H, t, J = 7Hz), 2.05-2.60(2H, m), 2.19(3H, d, J = 6Hz), 3.15-3.50(2H, m), 3.80-4.15(2H, m), 4.34(2H, q, J = 7Hz), 4.50-4.80(1H, m), 6.34(1H, q, J = 6Hz), 7.55-7.80(1H, m), 7.84(1H, d, J = 13.5Hz).

Specific rotation $[\alpha]_D^{20}$ - 294.1 ° (c = 0.1, $CHCl_3$)

The retention time of this product was 15.4 minutes under the following HPLC conditions:

| | |
|---|---|
| COLUMN: | SUMIPAX OA-3100 $\phi$ 4.6mm × 250mm |
| Carrier: | methanol |
| Flow rate: | 0.5ml/min |
| Detector: | UV spectrometer(287nm) |

Example 2

Ethyl 8-chloro-6-fluoro-1-methyl-4-oxo-7-((S)-3-trifluoroacetylamino-1-pyrrolidinyl)-1H,4H-[1,3]thiazeto[3,2-a]-quinoline-3-carboxylate (isomer B)

Ethyl 8-chloro-6-fluoro-1-methyl-4-oxo-7-((S)-3-trifluoroacetylamino-1-pyrrolidinyl)-1H,4H-[1,3]thiazeto-[3,2-a]quinoline-3-carboxylate (129 g) which is a diastereomeric mixture of isomers A and B was dissolved in 400 ml of hot isopropanol. The insoluble materials were filtered off. After the filtrate was cooled with ice water bath for 45 minutes, pale yellow crystals were filtered off. After the mother liquor was kept to stand, the precipitates were collected by suction to give 93.7 g of yellow crystals. Further, the mother liquor was evaporated to give a viscous brown liquid. The yellow crystals obtained above were dissolved in 350 ml of hot isopropanol. After cooling, the precipitates were filtered off and the mother liquor was evaporated. The residue and the viscous brown liquid obtained above were dissolved in hot ethyl acetate. After cooling, the precipitates were filtered off and the mother liquor was evaporated. The residue was recrystallized twice from isopropanol to give 2.43 g of the desired compound as colorless needles, m.p. 161-164. 5 °C (isomer B).

IR spectrum $\nu$ (KBr)$cm^{-1}$ : 1716, 1678, 1602.

NMR spectrum $\delta$ ($CDCl_3$)ppm: 1.39(3H, t, J = 7Hz), 1.90-2.68(2H, m), 2.22(3H, d, J = 6Hz), 3.36-3.88(4H, m), 4.36(2H, q, J = 7Hz), 4.18-4.80(1H, m), 6.34(1H, q, J = 6Hz), 7.12-7.44(1H, m), 7.93(1H, d, J = 13Hz).

Specific rotation $[\alpha]_D^{20}$ + 219.1 ° (c = 0.1, $CHCl_3$)

The retention time of this product was 17.5 minutes under the following HPLC conditions:

| | |
|---|---|
| COLUMN: | SUMIPAX OA-3100 $\phi$ 4.6mm × 250mm |
| Carrier: | methanol |
| Flow rate: | 0.5ml/min |
| Detector: | UV spectrometer(287nm) |

Example 3

Ethyl 8-chloro-6-fluoro-1-methyl-4-oxo-7-((R)-3-trifluoroacetylamino-1 -pyrrolidinyl)-1H,4H-[1,3]thiazeto[3,2-a]quinoline-3-carboxylate (isomer A)

Ethyl 8-chloro-6-fluoro-1-methyl-4-oxo-7-((R)-3-trifluoroacetylamino-1-pyrrolidinyl)-1H,4H-[1,3]thiazeto-[3,2-a]quinoline-3-carboxylate (30.2 g) which is a diastereomeric mixture of isomers A and B was dissolved

in hot ethyl acetate. After cooling, the precipitates were filtered off and the mother liquor was evaporated. The residue was recrystallized three times from isopropanol to give 6.91 g of the desired compound as colorless needles, m.p. 192-193.5 °C (isomer A).

IR spectrum $\nu$ (KBr)$cm^{-1}$ : 1712, 1672, 1602.

NMR spectrum $\delta$ ($CDCl_3$)ppm: 1.39(3H, t, J=7Hz), 1.92-2.60(2H, m), 2.20(3H, d, J=6Hz), 3.13-3.60(2H, m), 3.72-4.16(2H, m), 4.35(2H, q, J=7Hz), 4.48-4.80(1H, m), 6.35(1H, q, J=6Hz), 7.48-7.76(1H, m), 7.86 (1H, d, J=13Hz).

Specific rotation $[\alpha]_D^{20}$ + 293.7 ° (c=0.1,$CHCl_3$)

The retention time of this product was 17.5 minutes under the following HPLC conditions:

| | |
|---|---|
| COLUMN: | SUMIPAX OA-3100 $\phi$ 4.6mm × 250mm |
| Carrier: | methanol |
| Flow rate: | 0.5ml/min |
| Detector: | UV spectrometer(287nm) |

Example 4

Ethyl 8-chloro-6-fluoro-1-methyl-4-oxo-7-((R)-3-trifluoroacetylamino-1-pyrrolidinyl)-1H,4H-[1,3]thiazeto[3,2-a]-quinoline-3-carboxylate (isomer B)

Ethyl 8-chloro-6-fluoro-1-methyl-4-oxo-7-((R)-3-trifluoroacetylamino-1-pyrrolidinyl)-1H,4H-[1,3]thiazeto-[3,2-a]quinoline-3-carboxylate (45.5 g) which is a diastereomeric mixture of isomers A and B was dissolved in hot ethyl acetate. After cooling, the precipitates were collected by suction. The obtained crystals were recrystallized three times from isopropanol to give 8.70 g of the desired compound as colorless needles, m.p. 161-165 °C (isomer B).

IR spectrum $\nu$ (KBr)$cm^{-1}$ : 1716, 1676, 1604.

NMR spectrum $\delta$ ($CDCl_3$)ppm: 1.39(3H, t, J=7Hz), 1.81-2.70(2H, m), 2.22(3H, d, J=6Hz), 3.35-3.94(4H, m), 4.36(2H, q, J=7Hz), 4.19-4.81 (1H, m), 6.34(1H, q, J=6Hz), 7.10-7.43(1H, m), 7.92(1H, d, J=13Hz).

Specific rotation $[\alpha]_D^{20}$ - 213.2 ° (c=0.1,$CHCl_3$)

The retention time of this product was 15.4 minutes under the following HPLC conditions:

| | |
|---|---|
| COLUMN: | SUMIPAX OA-3100 $\phi$ 4.6mm × 250mm |
| Carrier: | methanol |
| Flow rate: | 0.5ml/min |
| Detector: | UV spectrometer(287nm) |

Example 5

Ethyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate (isomer A)

Ethyl 6,8-difluoro-1-methyl-4-oxo-7-((S)-3-trifluoroacetylamin o-1-pyrrolidinyl)-1H,4H-[1,3]thiazeto[3,2-a]-quinoline-3-carboxylate (68 g) which is a diastereomeric mixture of isomers A and B was recrystallized from a mixture of dichloromethane and isopropanol (1:4) to give 24.8 g of pale brown needles enriched with isomer A. The obtained crystals were recrystallized five times from a mixture of dichloromethane and isopropanol (1:4) and then once from acetonitrile to give 1.1 g of the desired compound as colorless plates, m.p. 239-242 °C (isomer A) .

IR spectrum $\nu$ (KBr)$cm^{-1}$ : 1716, 1672, 1636, 1604.

NMR spectrum $\delta$ ($CDCl_3$ )ppm: 1.35(3H, t, J=7Hz), 1.64-2.47(2H, m), 2.00(3H, dd, J=6,2Hz), 3.23-5.02-(5H, m), 4.31(2H, q, J=7Hz), 5.91 (1H, qd, J=6,2.5Hz), 7.21(1H, dd, J=14.5,2Hz) 9.44-9.97(1H, m).

Specific rotation $[\alpha]_D^{20}$ - 394.0 ° (c=0.1,$CHCl_3$)

The retention time of this product was 8.2 minutes under the following HPLC conditions:

| | |
|---|---|
| COLUMN: | CHIRALPAK AS $\phi$ 4.6mm × 250mm |
| Carrier: | n-hexane:ethanol (7:3) |
| Flow rate: | 1.0ml/min |
| Detector: | UV spectrometer(288nm) |

Example 6

Ethyl 6,8-difluoro-1-methyl-4-oxo-7-((S)-3-trifluoroacetylamino-1-pyrrolidinyl)-1H,4H-[1,3]thiazeto[3,2-a]-

quinoline-3-carboxylate (isomer B)

Ethyl 6,8-difluoro-1-methyl-4-oxo-7-((S)-3-trifluoroacetylam ino-1-pyrrolidinyl)-1H,4H-[1,3]thiazeto[3,2-a]-quinoline-3-carboxylate (68 g) which is a diastereomeric mixture of isomers A and B was recrystallized from a mixture of dichloromethane and isopropanol (1:4) and the precipitates were filtered off. The mother liquor was evaporated to give 40 g of pale brown crystals enriched with isomer B. The obtained crystals were recrystallized five times from 1,2-dichloroethane and then once from acetonitrile to give 0.35 g of the desired compound as colorless needles, m.p. 249-251 °C (isomer B).

IR spectrum $\nu$ (KBr)$cm^{-1}$ : 1732, 1712, 1636, 1606.

NMR spectrum $\delta$ ($CDCl_3$)ppm: 1.35(3H, t, J=7Hz), 1.79-2.45(2H, m), 2.05(3H, dd, J=7, 2.5Hz), 3.61-4.17(4H, m), 4.32(2H, q, J=7Hz), 4.52-4.78(1H, m), 5.86(1H, qd, J=7, 2.5Hz), 7.37(1H, dd, J=14.5, 2Hz) 8.68-8.96(1H, m).

Specific rotation $[\alpha]_D^{20}$ + 153.6 ° (c=0.1,$CHCl_3$)

The retention time of this product was 13.0 minutes under the following HPLC conditions:

| | |
|---|---|
| COLUMN: | CHIRALPAK AS $\phi$ 4.6mm × 250mm |
| Carrier: | n-hexane:ethanol (7:3) |
| Flow rate: | 1.0ml/min |
| Detector: | UV spectrometer(288nm) |

Example 7

Ethyl 6,8-difluoro-1-methyl-4-oxo-7-((R)-3-trifluoroacetylamino-1-pyrrolidinyl)-1H,4H-[1,3]thiazeto[3,2-a]-quinoline-3-carboxylate (isomer A)

Ethyl 6,8-difluoro-1-methyl-4-oxo-7-((R)-3-trifluoroacetylamin o-1-pyrrolidinyl)-1H,4H-[1,3]thiazeto[3,2-a]-quinoline-3-carboxylate (70 g) which is a diastereomeric mixture of isomers A and B was recrystallized ten times from a mixture of dichloromethane and isopropanol (1:4) to give the desired compound (isomer A) as colorless needles, m.p. 236-239 °C .

IR spectrum $\nu$ (KBr)$cm^{-1}$ : 1716, 1670, 1636.

NMR spectrum $\delta$ ($CDCl_3$)ppm: 1.35(3H, t, J=7Hz), 2.01(3H, dd, J=6, 2.5Hz), 2.07-2.27(2H, m), 3.52-3.67(1H, m), 3.75-3.86(1H, m), 3.91-4.19(2H, m),4.32(2H, q, J=7Hz), 4.74(1H, br-s), 5.92(1H, qd, J=6, 2.5Hz), 7.26(1H, dd, J=14, 1.5Hz), 9.58(1H, d, J=7.5Hz).

Specific rotation $[\alpha]_D^{20}$ + 392.6 ° (c=0.1,$CHCl_3$).

The retention time of this product was 15.4 minutes under the following HPLC conditions:

| | |
|---|---|
| COLUMN: | CHIRALPAK AS $\phi$ 4.6mm × 250mm |
| Carrier: | n-hexane:ethanol (7:3) |
| Flow rate: | 1.0ml/min |
| Detector: | UV spectrometer(288nm) |

Example 8

Ethyl 6,8-difluoro-1-methyl-4-oxo-7-((R)-3-trifluoroacetylamino-1-pyrrolidinyl)-1H,4H-[1,3]thiazeto[3,2-a]-quinoline-3-carboxylate (isomer B)

Ethyl 6,8-difluoro-1-methyl-4-oxo-7-((R)-3-trifluoroacetylamin o-1-pyrrolidinyl)-1H,4H-[1,3]thiazeto[3,2-a]-quinoline-3-carboxylate (70 g) which is a diastereomeric mixture of isomers A and B was recrystallized six times from 1,2-dichloroethane to give the desired compound (isomer B) as colorless crystals, m.p. 247-249 °C .

IR spectrum $\nu$ (KBr)$cm^{-1}$ : 1732, 1712, 1636.

NMR spectrum $\delta$ ($CDCl_3$)ppm: 1.35(3H, t, J=7Hz), 2.00-2.38(2H, m), 2.05(3H, dd, J=6, 2.5Hz), 3.70-4.07(4H, m), 4.31(2H, q, J=7Hz), 4.58(1H, br-s), 5.84(1H, qd, J=6, 2.5Hz), 7.34(1H, d, J=14.5Hz), 8.96(1H, d, J=6Hz).

Specific rotation $[\alpha]_D^{20}$ - 160.8 ° (c=0.1,$CHCl_3$).

The retention time of this product was 10.7 minutes under the following HPLC conditions:

| | |
|---|---|
| COLUMN: | CHIRALPAK AS $\phi$ 4.6mm × 250mm |
| Carrier: | n-hexane:ethanol (7:3) |
| Flow rate: | 1.0ml/min |
| Detector: | UV spectrometer(288nm) |

Example 9

Ethyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate (isomer A)

Ethyl 6,8-difluoro-1-methyl-4-oxo-7-((S)-3-trifluoroacetylamin o-1-pyrrolidinyl)-1H,4H-[1,3]thiazeto[3,2-a]-quinoline-3-carboxylate (20 g) which is a diastereomeric mixture of isomers A and B was chromatographed on silica gel using a mixture of ethyl acetate-n-hexane (2:1) as an eluent to give 4.7 g of the desired compound (isomer A), which was recrystallized from acetonitrile to give colorless plates, m.p. 239-242 °C. The obtained compound was consistent with that of Example 5.

Example 10

Ethyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate (isomer B)

Ethyl 6,8-difluoro-1-methyl-4-oxo-7-((S)-3-trifluoroacetylamin o-1-pyrrolidinyl)-1H,4H-[1,3]thiazeto[3,2-a]-quinoline-3-carboxylate (20 g) which is a diastereomeric mixture of isomers A and B was chromatographed on silica gel using a mixture of ethyl acetate-n-hexane (2:1) as an eluent to give 3.9 g of the desired compound (isomer B), which was recrystallized from acetonitrile to give colorless needles, m.p. 249-251 °C. The obtained compound was consistent with that of Example 6.

Example 11

Propyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate (isomer A)

Propyl 6,8-difluoro-1-methyl-4-oxo-7-((S)-3-trifluoroacetylami no-1-pyrrolidinyl)-1H,4H-[1,3]thiazeto[3,2-a]quinoline-3-carboxylate (6.9 g) which is a diastereomeric mixture of isomers A and B was chromatographed on silica gel using a mixture of ethyl acetate-n-hexane (2:3) as an eluent to give 2.4 g of the desired compound (isomer A), which was recrystallized from ethanol to give colorless needles, m.p. 242-243 °C (decomp.).

IR spectrum $\nu$ (KBr)$cm^{-1}$ : 1722, 1670, 1638.

NMR spectrum $\delta$ ($CDCl_3$)ppm: 1.02(3H, t, J = 7Hz), 1.72(2H, sestet, J = 7Hz), 2.01(3H, dd, J = 6, 2.5Hz), 2.08-2.28(2H, m), 3.48-4.29(6H, m), 4. 65-4.78(1H, m), 5.93(1H, qd, J = 6, 2.5Hz), 7.27(1H, dd, J = 14, 1.5Hz), 9.52(1H, d, J = 7.5Hz).

Specific rotation $[\alpha]_D^{20}$ - 367 ° (c = 0.1,$CHCl_3$).

The retention time of this product was 10.3 minutes under the following HPLC conditions:

| | |
|---|---|
| COLUMN: | CHIRALPAK AS $\phi$ 4.6mm × 250mm |
| Carrier: | n-hexane:ethanol (7:3) |
| Flow rate: | 1.0ml/min |
| Detector: | UV spectrometer(288nm) |

Example 12

Isopropyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate (isomer A)

Isopropyl 6,8-difluoro-1-methyl-4-oxo-7-((S)-3-trifluoroacetyla mino-1-pyrrolidinyl)-1H,4H-[1,3]thiazeto-[3,2-a]quinoline-3-carboxylate (17.1 g) which is a diastereomeric mixture of isomers A and B was chromatographed on silica gel using a mixture of ethyl acetate-n-hexane (1:2) as an eluent to give 1.4 g of the desired compound (isomer A), which was recrystallized from ethanol to give colorless prisms, m.p. 250-252 °C (decomp.).

IR spectrum $\nu$ (KBr)$cm^{-1}$ : 1716, 1666, 1638.

NMR spectrum $\delta$ ($CDCl_3$)ppm: 1.33(6H, d, J = 6Hz), 2.00(3H, dd, J = 6. 5, 2.5Hz), 2.06-2.28(2H, m), 3.49-4.18(4H, m), 4.67-4.78(1H, m), 5.21(1H, heptet, J = 6Hz), 5.90(1H, qd, J = 6.5, 3Hz), 7.23(1H, dd, J = 14, 1.5Hz), 9.70(1H, d, J = 7.5Hz).

Specific rotation $[\alpha]_D^{20}$ - 424 ° (c = 0.1,$CHCl_3$).

The retention time of this product was 14.9 minutes under the following HPLC conditions:

COLUMN: ULTRON ES OVM $\phi$ 4.6mm × 150mm
Carrier: acetonitrile : pH 3.5 phosphate buffer (15:85)
Flow rate: 1.0ml/min
Detector: UV spectrometer(288nm)

Example 13

Butyl 6,8-difluoro-1-methyl-4-oxo-7-((S)-3-trifluoroacetylamino-1-pyrrolidinyl)-1H,4H-[1,3]thiazeto[3,2-a]-quinoline-3-carboxylate (isomer A)

Butyl 6,8-difluoro-1-methyl-4-oxo-7-((S)-3-trifluoroacetylamin o-1-pyrrolidinyl)-1H,4H-[1,3]thiazeto[3,2-a]-quinoline-3-carboxylate (18.6 g) which is a diastereomeric mixture of isomers A and B was chromatographed on silica gel using a mixture of ethyl acetate-n-hexane (1:2) as an eluent to give 2.8 g of the desired compound (isomer A), which was recrystallized from isopropanol to give colorless needles, m.p. 222-223.5°C.

IR spectrum $\nu$ (KBr)$cm^{-1}$ : 1722, 1670, 1638.

NMR spectrum $\delta$ ($CDCl_3$)ppm: 0.98(3H, t, J=7.5Hz), 1.45(2H, sestet, J=7.5Hz), 1.58-1.82(2H, m), 2.01-(2H, dd, J=6, 2.5Hz), 2.08-2.29(2H, m), 3.48-4.18(4H, m), 4.18-4.34(2H, m), 4.65-4.80 (1H, m), 5.92(1H, qd, J=6, 2.5Hz), 7.27(1H, d, J=14Hz), 9.48(1H, d, J=7Hz).

Specific rotation $[\alpha]_D^{20}$ - 353 ° (c=0.1,$CHCl_3$).

The retention time of this product was 10.1 minutes under the following HPLC conditions:

COLUMN: CHIRALPAK AS $\phi$ 4.6mm × 250mm
Carrier: n-hexane:ethanol (7:3)
Flow rate: 1.0ml/min
Detector: UV spectrometer(288nm)

Example 14

7-((S)-3-Amino-1-pyrrolidinyl)-8-chloro-6-fluoro-1-methyl-4-oxo-1H,4H-[1,3]thiazeto[3,2-a]quinoline-3-carboxylic acid (isomer A)

A mixture of 0.50 g of ethyl 8-chloro-6-fluoro-1-methyl-4-oxo-7-((S)-3-trifluoroacetylamino-1-pyrrolidinyl)-1H,4H-[1,3]thiazeto[3,2-a]quinoline-3-carboxylate [isomer B, $[\alpha]_D^{20}$ +219.1° (c=0.1,$CHCl_3$)], the compound of Example 2, 0.28 g of 85 % potassium hydroxide, 1 ml of tert-butanol and 9 ml of water was heated at 60 °C for 1 hour with stirring. After cooling, the pH of the reaction mixture was adjusted to 8 with 10 % acetic acid. The precipitates were collected by suction to give 0.38 g of colorless crystals, which were converted to the p-toluenesulfonic acid salt in a usual manner. The salt was recrystallized from methanol to give 0.12 g of colorless needles, m.p. 179-182°C.

IR spectrum $\nu$ (KBr)$cm^{-1}$ : 1704, 1618.

NMR spectrum $\delta$ (DMSO-$d_6$)ppm: 1.80-2.68(2H, m), 2.18(3H, d, J= 6Hz), 2.28(3H, s), 3.32-4.17(5H, m), 6. 69(1H, q, J=6Hz), 7.09(2H, d, J=8Hz), 7.51(2H, d, J=8Hz), 7.83(1H, d, J=13.5Hz).

Specific rotation $[\alpha]_D^{20}$ + 283.3 ° (c=0.1,MeOH)

The retention time of this product was 14.2 minutes under the following HPLC conditions:

COLUMN: TSKgel ODS-80$T_M$ $\phi$ 4.6mm × 150mm
Carrier: acetonitrile : pH2.5 phosphate buffer (1:4) containing 0.005M 1-pentanesulfonic acid sodium salt
Flow rate: 1.0ml/min
Detector: UV spectrometer(287nm)

Example 15

7-((S)-3-Amino-1-pyrrolidinyl)-8-chloro-6-fluoro-1-methyl-4-oxo-1H,4H-[1,3]thiazeto[3,2-a]cuinoline-3-carboxylic acid (isomer B)

A mixture of 24.0 g of ethyl 8-chloro-6-fluoro-1-methyl-4-oxo-7-((S)-3-trifluoroacetylamino-1-pyrrolidinyl)-1H,4H-[1,3]thiazeto[3,2-a]quinoline-3-carboxylate [isomer A, $[\alpha]_D^{20}$ -294.1° (c=0.1,$CHCl_3$)], the compound of Example 1, 13.3 g of 85 % potassium hydroxide, 48 ml of tert-butanol and 144 ml of water was heated at

60 °C for 1 hour with stirring. After cooling, the pH of the reaction mixture was adjusted to 8 with 10 % acetic acid. The precipitates were collected by suction to give 18.8 g of pale yellow crystals, which were converted to the p-toluenesulfonic acid salt in a usual manner. The salt was recrystallized from methanol to give pale yellow needles, m.p. 184-187 °C.

IR spectrum $\nu$ (KBr)$cm^{-1}$ : 1692, 1620.

NMR spectrum $\delta$ (DMSO-$d_6$)ppm: 1.91-2.40(2H, m), 2.18(3H, d, J= 6Hz), 2. 28(3H, s), 2.66-4.21(5H, m), 6. 69(1H, q, J=6Hz), 7.09(2H, d, J=8Hz), 7.51(2H, d, J=8Hz), 7.83(1H, d, J=13Hz).

Specific rotation $[\alpha]_D^{20}$ - 236.4 ° (c=0.1,MeOH)

The retention time of this product was 16.0 minutes under the following HPLC conditions:

| | |
|---|---|
| COLUMN: | TSKgel ODS-80T $_M$ $\phi$ 4.6mm × 150mm |
| Carrier: | acetonitrile : pH2.5 phosphate buffer (1:4) containing 0.005M 1-pentanesulfonic acid sodium salt |
| Flow rate: | 1.0ml/min |
| Detector: | UV spectrometer(287nm) |

Example 16

7-((R)-3-Amino-1-pyrrolidinyl)-8-chloro-6-fluoro-1-methyl-4-oxo-1H,4H-[1,3]thiazeto[3,2-a]quinoline-3-carboxylic acid (isomer A)

A mixture of 2.00 g of ethyl 8-chloro-6-fluoro-1-methyl-4-oxo-7-((R)-3-trifluoroacetylamino-1-pyrrolidinyl)-1H,4H-[1,3]thiazeto[3,2-a]quinoline-3-carboxylate [isomer A, $[\alpha]_D^{20}$ +293.7° (c=0.1,$CHCl_3$)], the compound of Example 3, 1.10 g of 85 % potassium hydroxide, 4 ml of tert-butanol and 12 ml of water was heated at 60 °C for 1 hour with stirring. After cooling, the pH of the reaction mixture was adjusted to 8 with 10 % acetic acid. The precipitates were collected by suction to give 1.47 g of pale yellow crystals, which were converted to the p-toluenesulfonic acid salt in a usual manner. The salt was recrystallized from methanol to give pale yellow needles, m.p. 184-185.5 °C.

IR spectrum $\nu$ (KBr)$cm^{-1}$ : 1690, 1620.

NMR spectrum $\delta$ (DMSO-$d_6$)ppm: 1.82-2.64(2H, m), 2.18(3H, d, J= 6Hz), 2.28(3H, s), 2.86-4.22(5H, m), 6.68(1H, q, J=6Hz), 7.10(2H, d, J=8Hz), 7.51(2H, d, J=8Hz), 7.81(1H, d, J=13.5Hz).

Specific rotation $[\alpha]_D^{20}$ + 237.4 ° (c=0.1,MeOH)

The retention time of this product was 16.0 minutes under the following HPLC conditions:

| | |
|---|---|
| COLUMN: | TSKgel ODS-80T $_M$ $\phi$ 4.6mm × 150mm |
| Carrier: | acetonitrile : pH2.5 phosphate buffer (1:4) containing 0.005M 1-pentanesulfonic acid sodium salt |
| Flow rate: | 1.0ml/min |
| Detector: | UV spectrometer(287nm) |

Example 17

7-((R)-3-Amino-1-pyrrolidinyl)-8-chloro-6-fluoro-1-methyl-4-oxo-1H,4H-[1,3]thiazeto[3,2-a]quinoline-3-carboxylic acid (isomer B)

A mixture of 5.00 g of ethyl 8-chloro-6-fluoro-1-methyl-4-oxo-7-((R)-3-trifluoroacetylamino-1-pyrrolidinyl)-1H,4H-[1,3]thiazeto[3,2-a]quinoline-3-carboxylate [isomer B, $[\alpha]_D^{20}$ -213.2° (c=0.1,$CHCl_3$)], the compound of Example 4, 2.76 g of 85 % potassium hydroxide, 10 ml of tert-butanol and 30 ml of water was heated at 60 °C for 1.5 hours with stirring. After cooling, the pH of the reaction mixture was adjusted to 8 with 10 % acetic acid. The precipitates were collected by suction to give 2.17 g of colorless crystals, which were converted to the p-toluenesulfonic acid salt in a usual manner. The salt was recrystallized from methanol to give 1.28 g of pale yellow crystals, m. p. 222-226 °C(decomp.).

IR spectrum $\nu$ (KBr)$cm^{-1}$ : 1712, 1616.

NMR spectrum $\delta$ (DMSO-$d_6$)ppm: 1.74-4.11(7H, m), 2.18(3H, d, J= 6Hz), 2.28(3H, s), 6.70(1H, q, J=6Hz), 7.09(2H, d, J=8Hz), 7.50(2H, d, J=8Hz), 7.85(1H, d, J=13.5Hz).

Specific rotation $[\alpha]_D^{20}$ - 277.3 ° (c=0.1,MeOH)

The retention time of this product was 14.2 minutes under the following HPLC conditions:

| | |
|---|---|
| COLUMN: | TSKgel ODS-80T M $\phi$ 4.6mm × 150mm |
| Carrier: | acetonitrile : pH2.5 phosphate buffer (1:4) containing 0.005M 1-pentanesulfonic acid sodium salt |

Flow rate: 1.0ml/min
Detector: UV spectrometer(287nm)

Example 18

7-((S)-3-Amino-1-pyrrolidinyl)-6,8-difluoro-1-methyl-4-oxo-1H,4H-[1,3]thiazeto[3,2-a]quinoline-3-carboxylic acid (isomer A)

A mixture of 7.0 g of ethyl 6,8-difluoro-1-methyl-4-oxo-7-((S) -3-trifluoroacetylamino-1-pyrrolidinyl)-1H,4H-[1,3]thiazeto[3,2-a]quin oline-3-carboxylate [isomer A, $[\alpha]_D^{20}$ -394.0° (c = 0.1, $CHCl_3$)], the compound of Example 5, 4.7 g of 85 % potassium hydroxide, 25 ml of tert-butanol and 73 ml of water was heated at 55 °C for 1.5 hours with stirring. After cooling, the pH of the reaction mixture was adjusted to 8 with 10 % hydrochloric acid. The precipitates were collected by suction to give 5.2 g of pale brown crystals, which were converted to the p-toluenesulfonic acid salt in a usual manner. The salt was recrystallized from a mixture of N,N-dimethylformamide and ethanol (1:1) to give pale brown crystals, m.p. 218-220 °C (decomp.).

IR spectrum $\nu$ (KBr)$cm^{-1}$ : 1700, 1634.

NMR spectrum $\delta$ (DMSO-$d_6$)ppm: 2.09(3H, dd, J = 6, 3Hz), 2.27(3H, s), 1.81-2.40(2H, m), 3.43-4.19(5H, m), 6.38(1H, qd, J = 6, 2Hz), 7.08(2H, d, J = 8Hz), 7.49(2H, d, J = 8Hz), 7.59(1H, dd, J = 14, 2Hz).

Specific rotation $[\alpha]_D^{20}$ - 149.6 ° (c = 0.1,DMF)

The hydrochloride salt was obtained in a usual manner.

pale brown needles (MeOH), m.p. 233-235 °C (decomp.).

IR spectrum $\nu$ (KBr)$cm^{-1}$ : 1668, 1632.

NMR spectrum $\delta$ (DMSO-$d_6$)ppm: 2.10(3H, dd, J = 6, 3Hz), 1.84-2.36 (2H, m), 2.65-4.30(5H, m), 6.41(1H, qd, J = 6, 2.5Hz), 7.62(1H, dd, J = 14.5, 2Hz).

Specific rotation $[\alpha]_D^{20}$ - 272.0 ° (c = 0.1, $H_2O$)

The retention time of this product was 14.0 minutes under the following HPLC conditions:

COLUMN: TSKgel ODS-80T $_M$ $\phi$ 4.6mm × 150mm
Carrier: 6mM L-isoleucine and 3mM copper sulfate in water : methanol (4:1)
Flow rate: 1.0ml/min
Detector: UV spectrometer(287nm)

In the same manner as described in Example 18, the compounds of Examples from 19 to 22 were prepared.

Example 19

7-((S)-3-Amino-1-pyrrolidinyl)-6,8-difluoro-1-methyl-4-oxo-1H,4H-[1,3] thiazeto[3,2-a]quinoline-3-carboxylic acid (isomer A)

The desired compound was prepared using ethyl 6,8-difluoro-1-methyl-4-oxo-7-((S)-3-trifluoroacetylamino-1-pyrrolidinyl)-1H,4H-[1,3]thiazeto[3,2-a]quinoline-3-carboxylate [isomer A, $[\alpha]_D^{20}$ -394.0° (c = 0.1, $CHCl_3$)], the compound of Example 9. The obtained compound was consistent with that of Example 18.

Example 20

7-((S)-3-Amino-1-pyrrolidinyl)-6,8-difluoro-1-methyl-4-oxo-1H,4H-[1,3] thiazeto[3,2-a]quinoline-3-carboxylic acid (isomer A)

The desired compound was prepared using propyl 6,8-difluoro-1-methyl-4-oxo-7-((S)-3-trifluoroacetylamino-1-pyrrolidinyl)-1H,4H-[1,3] thiazeto[3,2-a]quinoline-3-carboxylate [isomer A, $[\alpha]_D^{20}$ -367 ° (c = 0.1, $CHCl_3$)], the compound of Example 11. The obtained compound was constent with that of Example 18.

Example 21

7-((S)-3-Amino-1-pyrrolidinyl)-6,8-difluoro-1-methyl-4-oxo-1H,4H-[1,3] thiazeto[3,2-a]quinoline-3-carboxylic acid (isomer A)

The desired compound was prepared using isopropyl 6,8-difluoro-1-methyl-4-oxo-7-((S)-3-trifluoroacetylamino-1-pyrrolidinyl)-1H,4H-[1,3]thiazeto[3,2-a]quinoline-3-carboxylate [isomer A, $[\alpha]_D^{20}$ - 424 ° (c=0.1,$CHCl_3$)], the compound of Example 12. The obtained compound was consistent with that of Example 18.

Example 22

<u>7-((S)-3-Amino-1-pyrrolidinyl)-6,8-difluoro-1-methyl-4-oxo-1H,4H-[1,3] thiazeto[3,2-a]quinoline-3-carboxylic acid (isomer A)</u>

The desired compound was prepared using butyl 6,8-difluoro-1-methyl-4-oxo-7-((S)-3-trifluoroacetylamino-1-pyrrolidinyl)-1H,4H-[1,3]thiazeto[3,2-a]quinoline-3-carboxylate [isomer A, $[\alpha]_D^{20}$ -353 ° (c=0.1,$CHCl_3$)], the compound of Example 13. The obtained compound was consistent with that of Example 18.

Example 23

<u>7-((S)-3-Amino-1-pyrrolidinyl)-6,8-difluoro-1-methyl-4-oxo-1H,4H-[1,3] thiazeto[3,2-a]quinoline-3-carboxylic acid (isomer B)</u>

A mixture of 5.0 g of ethyl 6,8-difluoro-1-methyl-4-oxo-7-((S) -3-trifluoroacetylamino-1-pyrrolidinyl)-1H,4H-[1,3]thiazeto[3,2-a]quin oline-3-carboxylate [isomer B, $[\alpha]_D^{20}$ +153.6° (c=0.1,$CHCl_3$)], the compound of Example 6, 3.4 g of 85 % potassium hydroxide, 18 ml of tert-butanol and 52 ml of water was heated at 55 °C for 1.5 hours with stirring. After cooling, the pH of the reaction mixture was adjusted to 8 with 10 % hydrochloric acid. The precipitates were collected by suction to give 3.7 g of pale brown crystals, which were converted to the p-toluenesulfonic acid salt in a usual manner. The salt was recrystallized from a mixture of ethanol and water (2:1) to give pale yellow needles, m.p. 217-219 °C(decomp.).

IR spectrum $\nu$ (KBr)$cm^{-1}$ : 1694, 1634.

NMR spectrum $\delta$ (DMSC-$d_6$)ppm: 2.08(3H, dd, 3=6, 3Hz), 2.27(3H, s), 1.81-2.40(2H, m), 3.53-4.19(5H, m), 6.39(1H, qd, 3=6, 2Hz), 7.08(2H, d, J=8Hz), 7.50(2H, d, J=8Hz), 7.58(1H, dd, J=14, 2Hz).

Specific rotation $[\alpha]_D^{20}$ + 109.1 ° (c=0.1,DMF)

The retention time of this product was 16.6 minutes under the following HPLC conditions:

| | |
|---|---|
| COLUMN: | TSKgel ODS-80T $_M$ $\phi$ 4.6mm × 150mm |
| Carrier: | 6mM L-isoleucine and 3mM copper sulfate in water : methanol (4:1) |
| Flow rate: | 1.0ml/min |
| Detector: | UV spectrometer(287nm) |

In the same manner as descrived in Example 23, the compound of Example 24 was prepared.

Example 24

<u>7-((S)-3-Amino-1-pyrrolidinyl)-6,8-difluoro-1-methyl-4-oxo-1H,4H-[1,3] thiazeto[3,2-a]quinoline-3-carboxylic acid (isomer B)</u>

The desired compound was prepared using ethyl 6,8-difluoro-1-methyl-4-oxo-7-((S)-3-trifluoroacetylamino-1-pyrrolidinyl)-1H,4H-[1,3]thiazeto[3,2-a]quinoline-3-carboxylate [isomer B, $[\alpha]_D^{20}$ + 153.6 ° (c=0.1,$CHCl_3$)], the compound of Example 10. The obtained compound was consistent with that of Example 23.

Example 25

<u>7-((R)-3-Amino-1-pyrrolidinyl)-6,8-difluoro-1-methyl-4-oxo-1H,4H-[1,3]thiazeto[3,2-a]quinoline-3-carboxylic acid (isomer A)</u>

A mixture of 3.0 g of ethyl 6,8-difluoro-1-methyl-4-oxo-7-((R) -3-trifluoroacetylamino-1-pyrrolidinyl)-1H,4H-[1,3]thiazeto[3,2-a]quin oline-3-carboxylate [isomer A, $[\alpha]_D^{20}$ +392.6° (c=0.1,$CHCl_3$)], the compound of Example 7, 2.0 g of 85 % potassium hydroxide, 10.5 ml of tert-butanol and 31.5 ml of water was heated at 55 °C for 1.5 hours with stirring. After cooling, the pH of the reaction mixture was adjusted to 8 with 10 % acetic acid. The precipitates were collected by suction to give 2.3 g of pale brown crystals, which

were converted to the p-toluenesulfonic acid salt in a usual manner. The salt was recrystallized from N,N-dimethylformamide to give pale yellow crystals, m.p. 218-220 °C (decomp.).

IR spectrum ν (KBr)$cm^{-1}$ : 1704, 1634.

NMR spectrum δ (DMSO-$d_6$)ppm: 2.09(3H, dd, J=6, 3Hz), 2.28(3H, s), 2.43-2.54(2H, m), 3.64-4.05(5H, m), 6.40 (1H, qd, J=6, 2.5Hz), 7.09(2H, d, J=8Hz), 7.48(2H, d, J=8Hz), 7.65(1H, dd, J=14.5, 2Hz).

Specific rotation $[\alpha]_D^{20}$ + 151 ° (c=0.1,DMF)

The retention time of this product was 18.7 minutes under the following HPLC conditions:

| | |
|---|---|
| COLUMN: | TSKgel ODS-80T $_M$ φ 4.6mm × 150mm |
| Carrier: | 6mM L-isoleucine and 3mM copper sulfate in water : methanol (4:1) |
| Flow rate: | 1.0ml/min |
| Detector: | UV spectrometer(287nm) |

Example 26

7-((R)-3-Amino-1-pyrrolidinyl)-6,8-difluoro-1-methyl-4-oxo-1H,4H-[1,3]thiazeto[3,2-a]quinoline-3-carboxylic acid (isomer B)

A mixture of 2.0 g of ethyl 6,8-difluoro-1-methyl-4-oxo-7-((R) -3-trifluoroacetylamino-1-pyrrolidinyl)-1H,4H-[1,3]thiazeto[3,2-a]quin oline-3-carboxylate [isomer B, $[\alpha]_D^{20}$ -160.8° (c=0.1,$CHCl_3$)], the compound of Example 8, 1.34 g of 85 % potassium hydroxide, 7 ml of tert-butanol and 21 ml of water was heated at 55 °C for 1.5 hours with stirring. After cooling, the pH of the reaction mixture was adjusted to 8 with 10 % acetic acid. The precipitates were collected by suction to give 1.55 g of pale brown crystals, which were converted to the p-toluenesulfonic acid salt in a usual manner. The salt was recrystallized from N,N-dimethylformamide to give pale yellow needles, m.p. 218-220 °C (decomp.).

IR spectrum ν (KBr)$cm^{-1}$ : 1698, 1634.

NMR spectrum δ (DMSO-$d_6$)ppm: 2.09(3H, dd, J=6, 3Hz), 2.28(3H, s), 2.50(2H, br-s), 3.64-4.06(5H, m), 6.41(1H, qd, J=6, 2Hz), 7.09(2H, d, J=8Hz), 7.49(2H, d, J=8Hz), 7.63(1H, dd, J=14.5, 0.5Hz).

Specific rotation $[\alpha]_D^{20}$ -103.8° (c=0.1,DMF)

The retention time of this product was 24.0 minutes under the following HPLC conditions:

| | |
|---|---|
| COLUMN: | TSKgel ODS-80T $_M$ φ 4.6mm × 150mm |
| Carrier: | 6mM L-isoleucine and 3mM copper sulfate in water : methanol (4:1) |
| Flow rate: | 1.0ml/min |
| Detector: | UV spectrometer(287nm) |

Example 27

Tablet formulation

| | |
|---|---|
| Compound of Example 18 | 110 mg |
| Lactose | q.s. |
| Corn starch | 34 mg |
| Magnesium stearate | 2 mg |
| Hydroxypropylmethylcellulose | 8 mg |
| Polyethyleneglycol 6000 | 0.5 mg |
| Titanium oxide | 0.5 mg |
| | 210 mg |

Example 28

Capsule formulation

| | |
|---|---|
| Compound of Example 18 | 110 mg |
| Lactose | q.s. |
| Carboxymethylcellulose | 15 mg |
| Hydroxypropylcellulose | 2 mg |
| Magnesium stearate | 2 mg |
| | 160 mg |

Example 29

Powder formulation

| | |
|---|---|
| Compound of Example 18 | 110 mg |
| Lactose | q.s. |

| | |
|---|---|
| D-Mannitol | 500 mg |
| Hydroxypropylcellulose | 5 mg |
| Talc | 2 mg |
| | 1000 mg |

Example 30

Injection formulation

| | |
|---|---|
| Compound of Example 18 | 50 mg |
| Glucose | 1000 mg |
| Hydrochloric acid | q.s. |
| Distilled water for injection | q.s. |
| | 20 ml |

Example 31

Suppository formulation

| | |
|---|---|
| Compound of Example 18 | 100 mg |
| Hard fat | 1300 mg |
| | 1400 mg |

## Claims

1. An optically active thiazetoquinoline-3-carboxylic acid compound represented by the following formula (I):

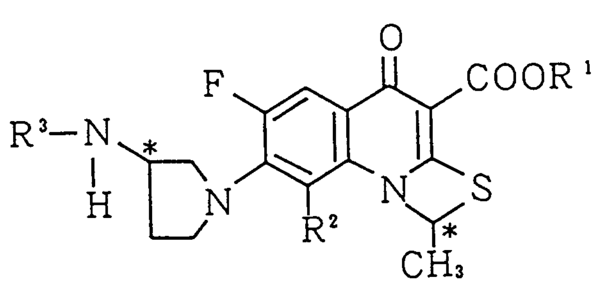

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl group, or the residue of carboxylic acid ester; and C * represents an asymmetric carbon atom of S- or R-configuration or a pharmacologically acceptable salt thereof.

2. An optically active thiazetoquinoline-3-carboxylic acid compound according to claim 1, wherein the

absolute configuration of the asymmetric carbon atom in the 3' position of the pyrrolidinyl group is S-configuration.

3. An optically active thiazetoquinoline-3-carboxylic acid compound according to claim 1, wherein the absolute configuration of the asymmetric carbon atom in the 3' position of the pyrrolidinyl group is R-configuration.

4. An optically active isomer of 7-((S)-3-amino-1-pyrrolidinyl)-8-chloro-6-fluoro-1-methyl-4-oxo-1H,4H-[1,3]-thiazeto[3, 3, 2-a]quinoline-3-carboxylic acid or a pharmacologically acceptable salt thereof where $[\alpha]_D^{20}$ of the p-toluenesulfonic acid salt of the free compound is about -236.4 ° in methanol at c = 0.1.

5. An optically active isomer of 7-((R)-3-amino-1-pyrrolidinyl)-8-chloro-6-fluoro-1-methyl-4-oxo-1H,4H-[1,3]-thiazeto[3, 2-a]quinoline-3-carboxylic acid or a pharmacologically acceptable salt thereof where $[\alpha]_D^{20}$ of the p-toluenesulfonic acid salt of the free compound is about -277.3 ° in methanol at c = 0.1.

6. An optically active isomer of 7-((S)-3-amino-1-pyrrolidinyl)- 6,8-difluoro-1-methyl-4-oxo-1H,4H-[1,3]-thiazeto[3,2-a]quinoline-3-carboxylic acid or a pharmacologically acceptable salt thereof where $[\alpha]_D^{20}$ of the p-toluenesulfonic acid salt of the free compound is about -149.6 ° in N,N-dimethylformamide at c = 0.1.

7. An optically active isomer of 7-((R)-3-amino-1-pyrrolidinyl)-6,8-difluoro-1-methyl-4-oxo-1H,4H-[1,3]-thiazeto[3,2-a]quinoline-3-carboxylic acid or a pharmacologically acceptable salt thereof where $[\alpha]_D^{20}$ of the p-toluenesulfonic acid salt of the free compound is about -103.8 ° in N,N-dimethylformamide at c = 0.1.

8. A process for preparing optically active thiazetoquinoline compound according to claim 1 comprising the steps of

(a) reacting an optically active aminopyrrolidinyl compound represented by the following formula (VII) having an asymmetric carbon atom of S- or R-configuration:

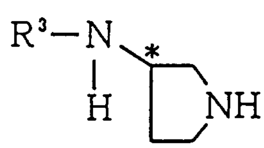

(VII)

wherein $R^3$ and C* are the same as those defined above, with racemic thiazetoquinoline compound represented by the following formula (VIII):

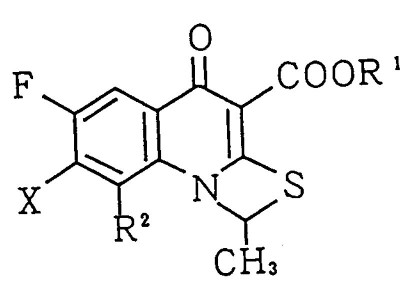

(VIII)

wherein $R^1$ and $R^2$ are the same as those defined above, and X represents a halogen atom, and followed by hydrolyzing if necessary, or chlorinating a thiazetoquinoline compound represented by the following formula (IX):

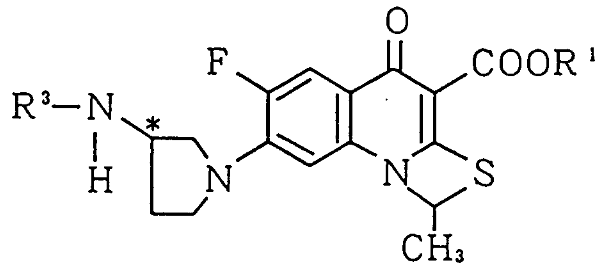

(XI)

wherein $R^1$, $R^2$, and C * are the same as those defined above, and followed by hydrolyzing if necessary to obtain a mixture of diastereoisomers,
(b) converting the mixture to a salt thereof, if necessary,
(c) fractionally recrystallizing or chromatographically separating the mixture of diastereoisomers to obtain an optically active compound, and
(d) hydrolysing the resulting optically active compound, if necessary.

**9.** An antibacterial agent comprising an optically active thiazetoquinoline-3-carboxylic acid compound represented by the following formula (I):

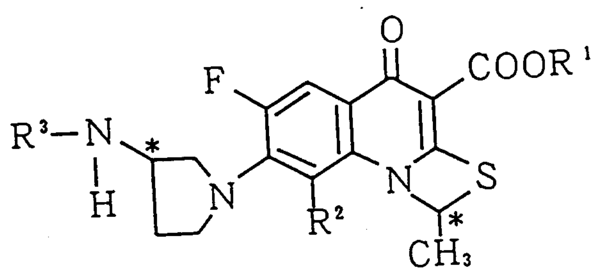

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl, or the residue of carboxylic acid ester; and C * represents an asymmetric carbon atom of S- or R-configuration or a pharmacologically acceptable salt thereof.

**10.** The pharmaceutical composition comprising an effective amount of one or more optically active thiazetoquinoline-3-carboxylic acid compound represented by the following formula (I):

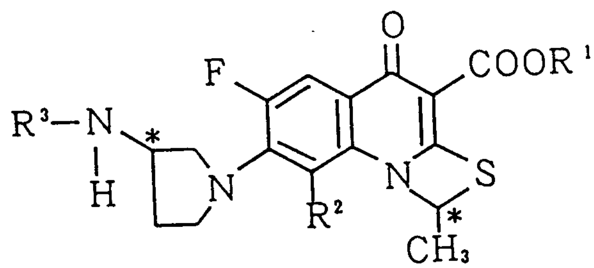

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl, or the residue of carboxylic acid ester; and C * represents an asymmetric carbon atom of S- or R-configuration or a pharmacologically acceptable salt thereof together with a pharmaceutically acceptable carrier or coating.

**11.** The pharmaceutical composition according to claim 10 useful for the treatment of bacterial infectious disease.

**12.** A method for the treatment of bacterial infectious diseases comprising the step of administering to an mammal an effective amount of an optically active thiazetoquinoline-3-carboxylic acid compound

represented by the following formula (I):

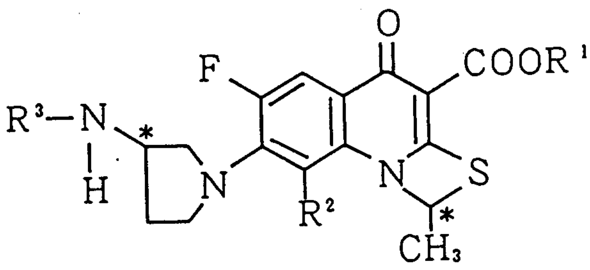

(I)

wherein $R^1$ is a hydrogen atom or a lower alkyl group; $R^2$ is a fluorine atom or a chlorine atom; $R^3$ is a hydrogen atom, a lower alkanoyl group, a halogenated lower alkanoyl, or the residue of carboxylic acid ester; and C * represents an asymmetric carbon atom of S- or R-configuration, a pharmacologically acceptable salt thereof, an antibacterial agent comprising the same, or a pharmaceutical composition comprising the same.

**13.** The method for the treatment of infectious diseases according to claim 12, wherein the mammal is a human being.

European Patent Office

# PARTIAL EUROPEAN SEARCH REPORT

which under Rule 45 of the European Patent Convention shall be considered, for the purposes of subsequent proceedings, as the European search report

Application Number: EP 90 11 9802

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | EP-A-0 315 827 (NIPPON SHINYAKU CO.) * The whole document, especially examples 1,2,9,10; page 18, lines 38-44 * | 1-11 | C 07 D 513/04<br>A 61 K 31/435 //<br>(C 07 D 513/04<br>C 07 D 285:00<br>C 07 D 221:00 ) |
| Y | EP-A-0 304 087 (WARNER-LAMBERT CO.) * Page 2, lines 1-4; page 13, lines 21-55; claims 1,8,12 * | 1-11 | |
| E | EP-A-0 393 538 (HOKURIKU PHARMACEUTICAL CO.) * The whole document * | 1-11 | |

TECHNICAL FIELDS SEARCHED (Int. Cl.5)

C 07 D 513/00
C 07 D 215/00
C 07 D 471/00
A 61 K 31/00

## INCOMPLETE SEARCH

The Search Division considers that the present European patent application does not comply with the provisions of the European Patent Convention to such an extent that it is not possible to carry out a meaningful search into the state of the art on the hasis of some of the claims

Claims searched completely : 1-11

Claims searched incompletely :

Claims not searched : 12,13

Reason for the limitation of the search:

Method for treatment of the human or animal body by surgery or therapy (see article 52(4) of the European Patent Convention).

| Place of search | Date of completion of the search | Examiner |
|---|---|---|
| THE HAGUE | 17-10-1991 | SEUFERT G.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document